# EUROPEAN PATENT SPECIFICATION

(11) **EP 2 499 258 B1**
(45) Date of publication and mention of the grant of the patent: **06.08.2014**
(21) Application number: 10776712.1
(22) Date of filing: 12.11.2010
(51) Int. Cl.: C12Q 1/68

(54) **SIGNATURE FOR THE DIAGNOSIS OF BREAST CANCER AGGRESSIVENESS AND GENETIC INSTABILITY**
Signatur für die Diagnose der Brustkrebsaggressivität und genetischen Instabilität
Signature pour le diagnostic de l'agressivité et l'instabilité génétique du cancer du sein

(30) Priority: 13.11.2009 EP 09306096
(43) Date of publication of application: 19.09.2012
(73) Proprietor: Centre National de la Recherche Scientifique (CNRS), 75016 Paris (FR); Université Paul Sabatier Toulouse III, 31400 Toulouse (FR); Institut Claudius Regaud, 31000 Toulouse (FR)
(72) Inventor: CAZAUX, Christophe, F-31830 Plaisance du Touch (FR); HOFFMANN, Jean-Sébastien, F-31500 Toulouse (FR); BOURDON, Jean-Christophe, Dundee DD2 1QD (GB); MACHADO DA SILVA, Alice, 30330-080 Belo Horizonte/MG (BR); ROCHE, Henri, F-31820 Pibrac (FR)
(74) Representative: Regimbeau
(86) International application number: PCT/EP2010/067401
(87) International publication number: WO 2011/058143

(56) References cited:
- WO-A1-2008/077165
- WANG XIANSHU ET AL: "Mutational analysis of thirty-two double-strand DNA break repair genes in breast and pancreatic cancers", CANCER RESEARCH, vol. 68, no. 4, February 2008 (2008-02), pages 971-975, XP002568121, ISSN: 0008-5472
- KAWAMURA KIYOKO ET AL: "DNA polymerase theta is preferentially expressed in lymphoid tissues and upregulated in human cancers.", INTERNATIONAL JOURNAL OF CANCER, vol. 109, no. 1, 10 March 2004 (2004-03-10), pages 9-16, XP002568122, ISSN: 0020-7136
- "Affymetrix GeneChip Human Genome U133 Array Set HG-U133A", GEO,, 11 March 2002 (2002-03-11), XP002254749, [retrieved on 2002-03-11]

## Description

### FIELD OF THE INVENTION

The present invention is in the field of breast cancer management, including diagnosis of aggressiveness and genetic instability of said breast cancer, and selection of an appropriate treatment. The invention is based on the finding that overexpression of POLQ is highly related to aggressiveness of a breast tumor, and thus to survival of the patient. The same overexpression is also correlated to genetic instability, which may then be used to kill tumor cells. Radiotherapy, for instance, is more efficacious on DNA which is already damaged. Likewise, DNA repair inhibitors may prevent repair of DNA breaks, thus leading to tumor cell death.

### BACKGROUND ART

Breast cancer is the most common malignancy among women and has one of the highest fatality rates of all cancers affecting females. Most breast cancers appear as a slowly growing, painless mass. There are a number of physical signs which might suggest the presence of breast cancer, and these may be discovered through a breast examination. Breast cancer metastasizes by direct extension, and via the lymphatics and the blood stream.

There are a number of methods currently used to treat breast cancer, including surgery, radiotherapy, hormone therapy, and chemotherapy. Successful cancer therapy is directed to the primary tumor and to any metastases, whether clinically apparent or microscopic. Because breast tumors may be cured with combined modality therapy, each of the above methods may be used alone, or in conjunction with one or more other therapies.

The selection of an appropriate treatment is crucial for the patient. It is essential to know when to use immediately a heavy and aggressive treatment protocol in order to prevent extension of an aggressive breast cancer. Otherwise, the survival of the patient may be highly compromised: although many techniques for detecting, diagnosing, and treating breast cancer exist, the disease remains the most common cancer in women, and one of the most lethal (Jemal et al., Cancer Statistics 59: 225-249, 2009). In contrast, performing a heavy and aggressive treatment when it is not necessitated by the tumor carried by the patient is also disadvantageous for the patient. Indeed, heavy and aggressive treatments always lead to adverse toxicities that may significantly affect the patient's quality of life. In addition, such heavy and aggressive treatments are usually very costly, and should thus be performed only when it is necessary.

Currently, treatment selection is based on tumor staging, which is usually performed using the Tumor/Node/Metastasis (TNM) test from the American Joint Committee on Cancer (AJCC). The TNM system assigns a number based on three categories. "T" denotes the tumor size, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome:
- Stage 0 describes noninvasive breast cancer. It has not spread within the breast or to other parts of the body.
- Stage I is an early stage of invasive breast cancer in which the tumor measures no more than 2 centimetres (cm) in diameter and no lymph nodes are involved. At this stage, the cancer has not spread outside the breast.
- Stage II, subdivided into IIA and IIB, describes invasive breast cancers in which one of the following is true:
   - The tumor measures less than 2 cm but has spread to lymph nodes under the arm
   - No tumor is found in the breast but cancer is found in the axillary lymph nodes
   - The tumor is between 2 cm and 5 cm (about 1 to 2 inches) and may have spread to lymph nodes under the arm
   - The tumor is larger than 5 cm but hasn't spread to any lymph nodes
- Stage III breast cancers are subdivided into three categories - IIIA, IIIB and III - based on a number of different criteria. By definition, stage III cancers haven't spread (metastasized) to distant sites.
- A stage IIIA tumor, for example, is larger than 5 cm and has spread to one to three lymph nodes under the arm. Other stage IIIA tumors may be any size and have spread into multiple lymph nodes. The lymph nodes clump together and attach to one another or to the surrounding tissue.
- In stage IIIB breast cancer, a tumor of any size has spread to tissues near the breast - the skin and chest muscles - and may have spread to lymph nodes within the breast or under the arm. Stage IIIB also includes inflammatory breast cancer, an uncommon but aggressive type of breast cancer.
- Stage IIIC cancer is a tumor of any size that has spread:
   - To 10 or more lymph nodes under the arm
   - To lymph nodes above or beneath the collarbone and near the neck
   - To lymph nodes within the breast itself and to lymph nodes under the arm
- Stage IV breast cancer has spread to other, distant parts of the body, such as the lungs, liver, bones or brain.

While this test and staging system provides some valuable information concerning the stage at which breast cancer has been diagnosed in the patient, it fails to identify the earliest stages of tumor progression. It has been shown that genetic instability is one of the first events of tumorigenesis (Bartkova et al., Nature, 434: 864-870, 2005; Gorgoulis et al., Nature, 434: 907-913, 2005). However, this cannot be detected with the TNM test, but only by assays which are difficult to use on a routine basis in the clinic. In addition, the TNM test does not give information on the tumor aggressiveness and its usefulness for prognosis is thus limited. Indeed, in order to make a reliable prognosis, it is important to be able to distinguish at the earliest stages between aggressive and non aggressive tumors. While it is clear that patients at stage IV have a bad prognosis, the fact that breast cancer is diagnosed at an early stage does not preclude the possibility that the tumor, although at its earliest stage, may further develop very rapidly. In contrast, when lymph nodes are invaded by tumor cells, the TNM test gives a bad prognosis and the patient is usually subjected to heavy surgery followed by intensive chemotherapy. However, if some of these patients were actually suffering from a non aggressive breast cancer, chemotherapy, which is an aggressive and mutagenic treatment, might be avoided without decreasing the survival prognosis of the patient.

There is thus a real need for better prognosis tests of breast cancer, not only to improve patient global survival, but also to improve their quality of life and to keep aggressive and costly chemotherapies for patients who will really benefit from them. The finding of new and more reliable prognosis tests is thus a very active research field.

In normal cell proliferation, DNA replication is performed by 3 DNA polymerases known as replicating polymerases (POLA, POLD and POLE). However, 10 other DNA polymerases have been identified in human cells, which are known as specialized polymerases and which functions are still largely unknown. These specialized polymerases appear to have been maintained through evolution because of their ability to process despite of DNA damage. It also appears that these polymerases are very mutagenic and that their activity is tightly controlled.

POLQ (also known as POL theta or POLθ) is one of these specialized DNA polymerases, and contains a helicase domain in its N-terminal portion and a polymerase domain in its C-terminus. Although the function of this particular specialized DNA polymerase is still largely unknown, it appears to be involved in maintenance of genome stability and in DNA repair (Seki et al., EMBO J, 23: 4484-4494, 2004; Masuda et al., Proc. Natl. Acad. Sci. U.S.A., 102: 13986-13991, 2005, Yoshimura et al., Mol. Cell, 24 : 115-125, 2006).

Kawamura et al (Kawamura et al., Int J Cancer, 109: 9-16, 2004) have compared the expression level of POLQ in several non-tumor tissues, including the mammary gland, and in lung (27 patients), colon (26 patients) and stomach (28 patients) cancer tissue. They found that POLQ is only barely detectable in non-tumor tissue, but that it is over-expressed in a significant proportion of lung, stomach and cancer tissues. The authors also indicate that over-expression of POLQ in tumor tissue is correlated to poor survival prognosis. However, they did not test for the level of POLQ expression in either breast tumor cell lines or breast tumors.

The inventors analyzed the variation of expression of the POLQ gene in tumor versus adjacent normal breast tissues and compared these data with disease progression and clinical features. They showed that POL Q was significantly overexpressed in breast tumor tissues. In addition, they demonstrated that POLQ deregulated expression contributed actively to tumor progression. Indeed, POLQ overexpression leads to genetic instability and notably DNA breaks.

### DESCRIPTION OF THE INVENTION

The present invention thus provides a method for diagnosing the aggressiveness of a breast cancer in a patient. According to the method of the invention, elevated expression levels of the POLQ gene indicate aggressiveness of said breast cancer.

Therefore, the present invention provides a method for diagnosing aggressiveness of a breast cancer in a patient from a breast cancer sample of said patient, comprising:
a) measuring in vitro the expression level of the POLQ gene and the expression level of a control gene in said patient breast cancer sample,
b) calculating for said POLQ gene an expression level ratio of the expression level of POLQ to the expression of the said control gene in said patient breast cancer sample,
c) comparing the said POLQ expression level ratio to a corresponding threshold value, and
d) diagnosing breast cancer aggressiveness if the said POLQ expression level ratio is superior to its corresponding threshold value.

In particular, in one embodiment, the method of the invention may be used for prognosing the survival of a patient with a high number of metastatic lymph nodes. In this embodiment, a high level of POLQ expression in a patient with a high number of metastatic lymph nodes indicates aggressiveness of the tumor, and results in poor survival prognosis, whereas low level of POLQ expression in a patient with a high number of invaded lymph nodes is associated to a much better diagnosis. A low number of metastatic lymph nodes may be defined, for example, as a invaded lymph node count equal to 1 or less, while a high number of metastatic lymph nodes may be defined as a invaded lymph node count equal to 2 or more.

In another embodiment, the method of the invention may be used for prognosing the survival of a patient which has a tumor expressing a p53^{wt} cDNA (the sequence does not contain any mutations by reference to sequence NC 000017-9 from GenBank). In this embodiment, a high level of POLQ expression in a patient with a tumor expressing a p53^{wt} cDNA indicates aggressiveness of the tumor, and results in poor survival prognosis, whereas low level of POLQ expression in a patient with a tumor expressing a p53^{wt} cDNA is associated to a much better diagnosis. Indeed, the inventors have shown that a low level of POLQ expression in a patient with a tumor expressing a p53^{wt} cDNA is associated with a probablity of survival of 98 %.

In another aspect, the method of the invention allows for the detection of cancer cells displaying genetic instability. Deregulated expression of POLQ leads to increased DNA damage and chromosome instability, thus triggering constitutive activation of the γH2AX-ATL-CHK2 DNA damage checkpoint.

Therefore, the present invention provides a method for diagnosing genetic instability in a breast cancer in a patient from a breast cancer sample of said patient, comprising:
a) measuring in vitro the expression level of the POLQ gene and the expression level of a control gene in said patient breast cancer sample,
b) calculating for said POLQ gene an expression level ratio of the expression level of POLQ to the expression of the said control gene in said patient breast cancer sample,
c) comparing the said POLQ expression level ratio to a corresponding threshold value, and
d) diagnosing breast cancer genetic instability if the said POLQ expression level ratio is superior to its corresponding threshold value.

It is understood that both methods can be combined. Therefore, in a further aspect, the invention relates to a method for diagnosing the aggressiveness of a breast cancer in a patient and for the detection of cancer cells displaying genetic instability. The method of the invention thus presents the advantage of allowing the detection of a genetic event associated with a bad survival prognosis and genetic instability.

As used herein, the term "POLQ" refers to the human gene encoding the DNA polymerase theta (Entrez Gene ID number: 10721; mRNA sequence reference: NM_199420.3; protein sequence reference: NP_955452.3). In addition, the invention encompasses all the isoforms of the said POLQ gene. Isoform, as used herein, refers to all the different forms of the POLQ gene and may be produced by mutations, or may arise from the same gene by alternative splicing. A large number of isoforms are caused by single nucleotide polymorphisms or SNPs, small genetic differences between alleles of the same gene. These occur at specific individual nucleotide positions within a gene. Also included within this definition is the situation where different versions of messenger RNA are created from the same gene by employing different promoters, which causes transcription to skip certain exons. Thus, it is understood that the methods of the invention are not restricted to POLQ per se, but also encompass one or several POLQ isoforms. According to methods of the invention, the level of the expression of the POLQ gene and/or one or several of its isoforms are measured, and ratios of expression are calculated.

According to the present invention, "aggressiveness" of a breast cancer is intended to mean the propensity of said breast cancer to invade the intestinal wall, lymph nodes and to generate metastases and the rapidity with which said invasions may appear. Aggressiveness of the breast cancer is obviously correlated to survival, and the above method may be used for prognosing survival of the patient, in which case diagnosing of aggressiveness results in a bad survival prognosis and diagnosis of the absence of aggressiveness results in a good survival prognosis.

As used herein, "genetic instability" of a breast cancer is intended to mean the propensity of tumor cells of said breast cancer to suffer from DNA damage. Genetic instability is the hallmark of breast cancer in which DNA damage are more frequent. By "DNA damage" is meant injury to DNA that affects the normal function of the DNA by causing covalent modification of the DNA or causing it to deviate from its normal double-helical conformation. DNA damage includes structural distortions which interfere with replication and transcription, as well as point mutations which can disrupt base pairs and can change the DNA sequence. In general, when a cell incurs DNA damage, the cell cycle is arrested at one of three checkpoints (Gl/S, intra-S or G2/M). The cell cycle arrest can lead to the activation of DNA repair processes (in the case of relatively minor DNA damage), or result in the induction of apoptosis (in the case of catastrophic DNA damage).

DNA damage can be caused spontaneously by endogenous processes such as oxidation of bases and generation of DNA strand interruptions by reactive oxygen and free radicals produced from normal metabolism, methylation of bases, depurination, depyrimidination, mismatch of bases by DNA polymerases during DNA replication, etc. DNA damage can also be caused by environmental insults such as radiation (e.g., ultraviolet radiation, x-rays, gamma rays, ionizing radiation), natural toxins (e.g., plant toxins), synthetic toxins, drugs (e.g., cancer chemotherapy, radiation therapy), alkylating agents, etc. DNA damage can lead to or result from a variety of disorders, including hereditary genetic disorders and disorders as a result of exposure to environmental insults. DNA damage can also occur as a result of smoking, leading to for example, heart disease, or as a result of therapies for other diseases, such as cancers.

The above methods are performed using a breast cancer sample of the patient to be tested. In some cases, the methods according to the invention may further comprise a preliminary step of taking a breast cancer sample from the patient. By a "breast cancer sample", it is referred to a tumor breast tissue sample. Even in a cancerous patient, breast tissue still comprises non tumor healthy tissue. The "breast cancer sample" should thus be limited to tumor breast tissue taken from the patient. Said "breast cancer sample" may be a biopsy sample or a sample taken from a surgical breast resection therapy.

In addition, the methods according to the invention may comprise another preliminary step, between the taking of the sample from the patient and steps a) as defined above, corresponding to the transformation of the breast cancer sample (and optionally of the healthy breast tissue sample) into a mRNA (or corresponding cDNA) sample or into a protein sample, which is then ready to use for in vitro measuring of genes expression levels in step a). Preparation or extraction of RNA (as well as retrotranscription into cDNA) or proteins from a tissue sample is only routine procedure well known to those skilled in the art.

Once a ready-to-use breast cancer mRNA (or corresponding cDNA) or protein sample is available, the measure of POLQ gene expression levels may be performed, depending on the type of transformation and the available ready-to-use sample, either at the mRNA (i.e. based on the mRNA content of the sample) or at the protein level (i.e. based on the protein content of the sample). In some embodiments, the expression levels of some of the genes may be measured at the mRNA level, while the expression levels of other genes are measured at the protein level. In this case, part of the breast cancer sample taken from the patient has been transformed into an mRNA (or corresponding cDNA) sample and another part has been transformed into a protein sample. In other embodiments, the expression levels of all tested genes are measured either at the mRNA or at the protein level.

When expression levels are measured at the mRNA level, it may be notably performed using well known technologies such as quantitative PCR or nucleic acid microarray technologies (including cDNA and oligonucleotide microarrays). These technologies are now used routinely by those skilled in the art and thus do not need to be detailed here. Examples of embodiments using quantitative PCR are described in the experimental section. Alternatively, any known or future technology permitting to assess genes expression levels based on mRNA contents may be used. For instance, tissue microarrays coupled to fluorescent in situ hybridization may be used. Tissue microarrays (also known as TMAs) consist of paraffin blocks in which up to 1000 separate tissue cores are assembled in array fashion to allow multiplex histological analysis. In the tissue microarray technique, a hollow needle is used to remove tissue cores as small as 0.6 mm in diameter from regions of interest in paraffin-embedded tissues such as clinical biopsies or tumor samples. These tissue cores are then inserted in a recipient paraffin block in a precisely spaced, array pattern. Sections from this block are cut using a microtome, mounted on a microscope slide and then analyzed by any method of standard histological analysis. Each microarray block can be cut into 100 - 500 sections, which can be subjected to independent tests. Tests commonly employed in tissue microarray include immunohistochemistry, and fluorescent in situ hybridization. For analysis at the mRNA level, tissue microarray technology may be coupled to fluorescent in situ hybridization.

When expression levels are measured at the protein level, it may be notably performed using specific antibodies, in particular using well known technologies such as western blot, ELISA or ELISPOT, antibodies microarrays, or tissue microarrays coupled to immunohistochemistry.

The comparison of the expression levels of the measured genes in said patient's breast cancer sample is made by calculating an expression level ratio of the expression level of the POLQ gene to the expression level of a control gene in said patient's breast cancer sample, and by comparing the obtained expression level ratio to a corresponding threshold value. Said control gene, according to the present invention, is a gene which is expressed in all cell types. More specifically, the control gene according to the invention is a gene which is expressed in all the cells constituting the breast. In another aspect, the expression level of the control gene is not affected by the state of the cell, i.e. the control gene is expressed to the same level in a healthy cell and in a tumor cell. In a specific embodiment, the control gene is a housekeeping gene. A housekeeping gene is a gene expressed in all cell types, which provides a basic function needed for sustenance of all cell types. A list of human housekeeping genes may be found in Eisenberg et al. (Trends in Genetics 19: 362-365, 2003). A preferred housekeeping gene according to the invention is a gene selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. A further preferred housekeeping gene according to the invention is IPO8 or HMBS. According to the present invention, a "threshold value" is intended to mean a value that permits to discriminate samples in which the expression level ratio of the gene of interest corresponds to an expression level of said gene of interest in the patient's breast cancer sample that is low or high. In particular, if a gene expression level ratio is inferior or equal to the threshold value, then the expression level of this gene in the patient's breast cancer sample is considered low, whereas if a gene expression level ratio is superior to the threshold value, then the expression level of this gene in the patient's breast cancer sample is considered high. For each gene, and depending on the method used for measuring the expression level of the genes, the optimal threshold value may vary. However, it may be easily determined by a skilled artisan based on the analysis of several control breast cancer samples in which the expression level (low or high) is known for this particular gene, and on the comparison thereof with the expression of a control gene, e.g. a housekeeping gene. In particular, the inventors determined that a unique threshold value of 15.8 is particularly useful. The inventors have shown that the patient survival is significantly decreased for expression level ratios above this value. This unique threshold value of 15.8 is specifically useful when the expression level of all genes is measured at the mRNA level using quantitative PCR.

The present invention further relates to a microarray dedicated to the implementation of the methods according to the invention, comprising at most 500, preferably at most 300, at most 200, more preferably at most 150, at most 100, even more preferably at most 75, at most 50, at most 40, at most 30, at most 20, at most 10 distinct probes, at least 1 of which specifically binds to POLQ mRNA (or corresponding cDNA) or protein.

In a preferred embodiment, said microarray is a nucleic acid microarray, comprising at most 500, preferably at most 300, at most 200, more preferably at most 150, at most 100, even more preferably at most 75, at most 50, at most 40, at most 30, at most 20, at most 10 distinct probes (thus excluding for instance pangenomic microarrays), at least 1 of which specifically hybridizes to POLQ mRNA (or corresponding cDNA). Said microarray may also contain at least one probe which specifically hybridizes to a housekeeping gene in addition to the probe specifically hybridizing to POLQ. In one embodiment, said housekeeping gene is selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. More preferentially, the housekeeping gene is the IPO8 or the HMBS gene. According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray", the oligonucleotides being about 25 to about 60 base pairs or less in length).

Alternatively, in another embodiment, said microarray may be an antibodies microarray, comprising at most 500, preferably at most 300, at most 200, more preferably at most 150, at most 100, even more preferably at most 75, at most 50, at most 40, at most 30, at most 20, at most 10 distinct antibodies, at least 1 of which specifically bind to POLQ protein. Said microarray may also contain at least one antibody which specifically binds to a housekeeping protein, in addition to the antibody specifically binding to the POLQ protein. In one embodiment, said housekeeping protein is selected in the group consisting of the B2M, TFRC, YWHAZ, RPLO, 18S, GUSB, UBC, TBP, GAPDH, PPIA, POLR2A, ACTB, PGK1, HPRT1, IPO8 and HMBS proteins. In a preferred embodiment, said housekeeping protein is the IPO8 or HMBS protein.

Alternatively to nucleic acid or antibody microarray technology, quantitative PCR may be used and amplification primers specific for the genes to be tested are thus also very useful for performing the methods according to the invention. The present invention thus further relates to a kit for diagnosing aggressiveness and genetic instability of a breast cancer in a patient from a breast cancer sample of said patient, comprising a dedicated microarray as described above or amplification primers specific for POLQ. Here also, when the kit comprises amplification primers, while said kit may comprise amplification primers specific for other genes, said kit preferably comprises at most 100, at most 75, 50, at most 40, at most 30, preferably at most 25, at most 20, at most 15, more preferably at most 10, at most 8, at most 6, even more preferably at most 5, at most 4, at most 3 or even 2 or one or even zero couples of amplification primers specific for other genes than POLQ. For example, said kit may comprise at least a couple of amplification primers for at least one housekeeping gene in addition to the primers for POLQ. In one embodiment, said housekeeping gene is selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. In a preferred embodiment, said housekeeping gene is the IPO8 or HMBS gene.

As mentioned above, the ability of prognosing breast cancer evolution, which is linked to its aggressiveness, is very important for selecting a suitable treatment, since heavy and costly treatments with potentially severe adverse effects should be used, in addition to traditional surgical treatment, each time they are necessary, but only when they are necessary. The present invention thus also concerns a method for choosing a suitable treatment of breast cancer in a patient, comprising:
a) diagnosing or not aggressiveness of said breast cancer in said patient using the method according to the invention as described above, and
b) adding adjuvant radiotherapy or chemotherapy to surgical treatment if said breast cancer is diagnosed as aggressive in step a).

In addition to its prognostic value concerning aggressiveness of breast cancer, the inventors also found that the same test based on the analysis of the expression levels of POLQ also allows diagnosing the presence or absence of genetic instability, thus resulting in the above described method for diagnosing genetic instability. In particular, the present inventors have shown that deregulated expression of POLQ is associated with an increased frequency of DNA breaks. In one preferred embodiment, the method of the invention is thus used to diagnose DNA breaks. Such genetic instability, and notably the increased frequency of DNA breaks, may have consequences concerning the selection of an adjuvant therapy. In particular, while genetic instability may favor tumor cells mutations and thus deregulation of proliferation and adhesion, the presence of DNA breaks might also be used against tumor cells. Indeed, for continued proliferation, those DNA breaks have to be repaired, and cells with a high number of DNA breaks are usually prone to cell death. As a result, while radiotherapy may not be efficient in all circumstances, its efficiency on tumor cells that already present an increased frequency of DNA breaks may be improved. For example, radiotherapy may be highly efficient against the POLQ-overexpressing breast tumor cells identified by the method of the invention, because these cells contain high levels of DNA breakage and chromosomal instability. In the same manner, the use of DNA repair inhibitors might permit to freeze DNA breaks and lead to tumor cells death.

More generally, the inventors have shown that the inhibition the DNA damage checkpoint (DDC) or DNA metabolism leads to a decreased viability of POLQ-overexpressing cells. For example, cells affected in the Chk2 kinase activity are unable to detect the POLQ overexpression-induced DNA lesions and/or stop the cell cycle in response to the said lesions. Likewise, POLQ-overexpressing cells show increased sensitivity to DDC or nucleotide metabolism inhibition.

The present invention thus also concerns a method for prognosing the efficiency of radiotherapy or DNA repair DNA damage signaling or nucleotide metabolim inhibitors in the treatment of breast cancer in a patient, comprising:
a) diagnosing or not genetic instability in said breast cancer in said patient using the method according to the invention as described above, and
b) prognosing efficiency of radiotherapy or DNA repair, DNA damage signaling or nucleotide metabolim inhibitors if genetic instability has been diagnosed in step a).

According to the invention, a "DNA repair inhibitor" is intended to mean a molecule that is able to inhibit repair of DNA breaks, in particular double stranded DNA breaks. While this expression should not be understood as limitative, examples of DNA repair inhibitors include inhibitors of DNA repair protein PARP (see e.g. WO 2004080976, WO 2005/053662, WO 2009/046205), inhibitors of histone deacetylase, such as those described in PCT application WO 2008/082856, and inhibitors of DNA polymeras β(see WO2007001684). A "DDC inhibitor" is a molecule which is capable of blocking the activity of any of the proteins involved in the DNA damage checkpoint. Examples of such proteins include ATM/ATR, Chk2 and Chk1. "Nucleotide metabolism inhibitors", as used herein, include all compounds which result in an imbalance of the nucleotide pools, because said nucleotide metabolism inhibitors are e.g. nucleotide analogues (ex: 6TG) or because they inhibit nucleotide biosynthesis enzymes (ex: HU).

In another embodiment, the present invention thus also covers a method for isolating new compounds capable of inhibiting DNA repair, DNA damage signaling or nucleotide metabolim, said method comprising the following steps:
a) contacting a compound with a cell overexpressing POLQ or with a cell which does not overexpress POLQ, and
b) assaying whether the compound affects the viability of the POLQ-overexpressing cell more than the viability of the cell not overexpressing POLQ.

The cell overexpressing POLQ can be any type of cell wherein the expression of POLQ is deregulated, whereas the cell which does not overexpress POLQ is a cell in which the regulation of the POLQ expression is maintained. For example, the said cell overexpressing POLQ can be obtained by transfection of a host cell with a backbone vector carrying the POLQ gene or cDNA under the control of a strong ubiquitous promoter. In this case, said cell is advantageously the said host cell transfected with the same backbone vector, wherein said vector does not contain the POLQ insert.

In yet another aspect, the invention also relates to the use of one or more DNA repair, DNA damage signaling or nucleotide metabolim inhibitors for the preparation of a medicament intended for the treatment of breast cancer in patients whose breast tumor over-expresses POLQ. According to the present invention, a gene is considered as "overexpressed" in a breast cancer sample if the ratio of the expression level of said gene to the expression level of a control gene in said breast cancer sample is superior to a threshold value as defined above. In a particular embodiment, said threshold value may be 15.8.

The present invention also relates to a method for treating a patient suffering from a breast cancer overexpressing POLQ, comprising subjecting said patient to radiotherapy and/or administering to said patient an effective amount of one ore more DNA repair inhibitors.

### FIGURES LEGENDS

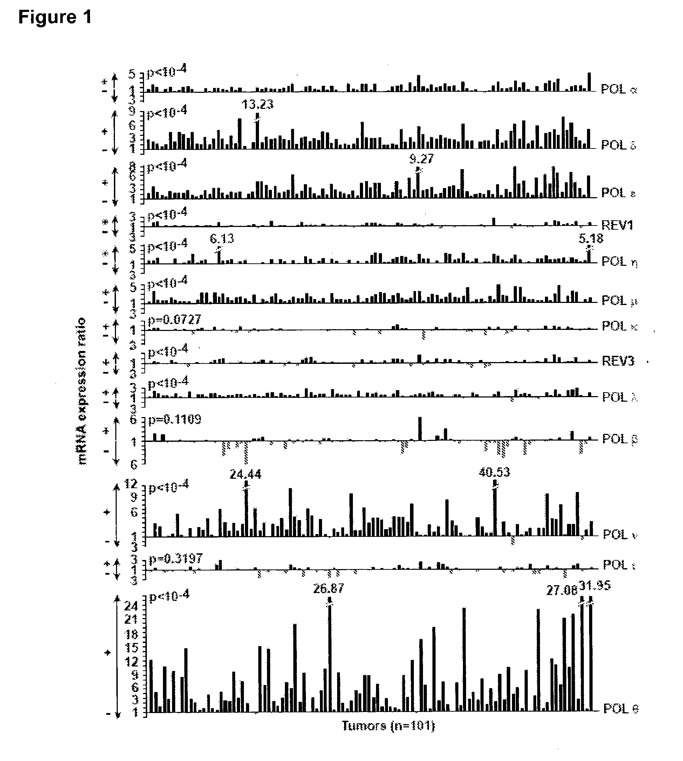

**Figure 1****. Relative expression of DNA polymerase genes in breast tumors from set 1 (France, n=105)**. mRNA expression ratios for tumor (T) and normal (N) breast samples (T/N) were-calculated (expression levels were previously normalized in relation to control genes). T/N>1 and <1 indicate higher and lower expression levels in tumors compared to pooled normal tissues, respectively. The p-values from the bilateral exact binomial test are given uncorrected; the significance level is evaluated using the Benjamini 2001 procedure for an overall FDR of 0.05. The patient samples indicated by the x axis are classified in the same order for every panel. + and - stand for higher and lower expression in the tumor (T) compared to the normal (N) tissues, respectively. For graph representation T/N values lower than 1 were transformed into the inverse N/T values.

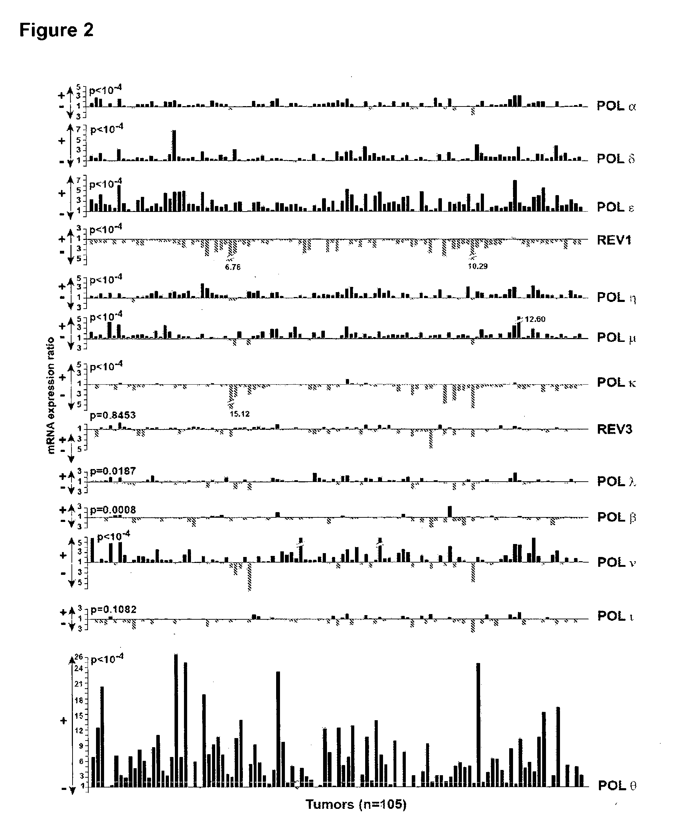

**Figure 2****. Relative expression of DNA polymerase genes in breast tumors from set 2 (France, n=101)**. mRNA expression ratios for tumor (T) and normal (N) breast samples (T/N) were calculated (expression levels were previously normalized in relation to control genes). T/N>1 and <1 indicate higher and lower expression levels in tumors compared to pooled normal tissues, respectively. The p-values from the bilateral exact binomial test are given uncorrected; the significance level is evaluated using the Benjamini 2001 procedure for an overall FDR of 0.05. The patient samples indicated by the x axis are classified in the same order for every panel. + and - stand for higher and lower expression in the tumor (T) compared to the normal (N) tissues, respectively. For graph representation T/N values lower than 1 were transformed into the inverse N/T values.

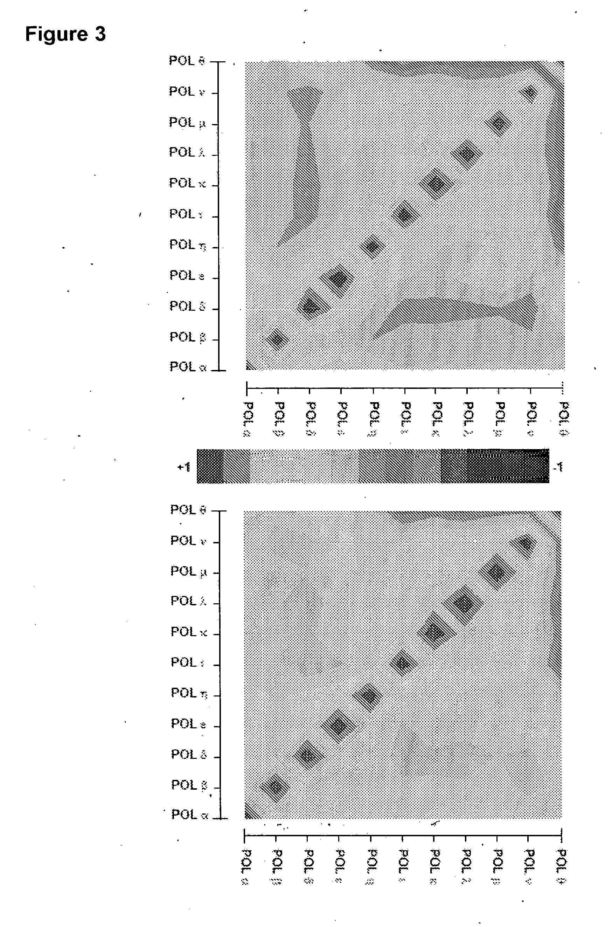

**Figure 3****. Gene to gene comparison of relative gene expression in tumors.** Graphical display of all pair wise correlations between the expression of the DNA polymerases in French breast tumors (Non parametric Spearman's correlation; (A) set 1 (n=105); (B) set 2 (n=101)). The closer to 1 the Spearman rho coefficient is (illustrated by a red or yellow zone at the intersection of horizontal and vertical axis), the greater the association between the expression of the two genes considered. Conversely, rho values lower than 1 (illustrated by a green zone) indicates that the expressions under comparison are independent from each other.

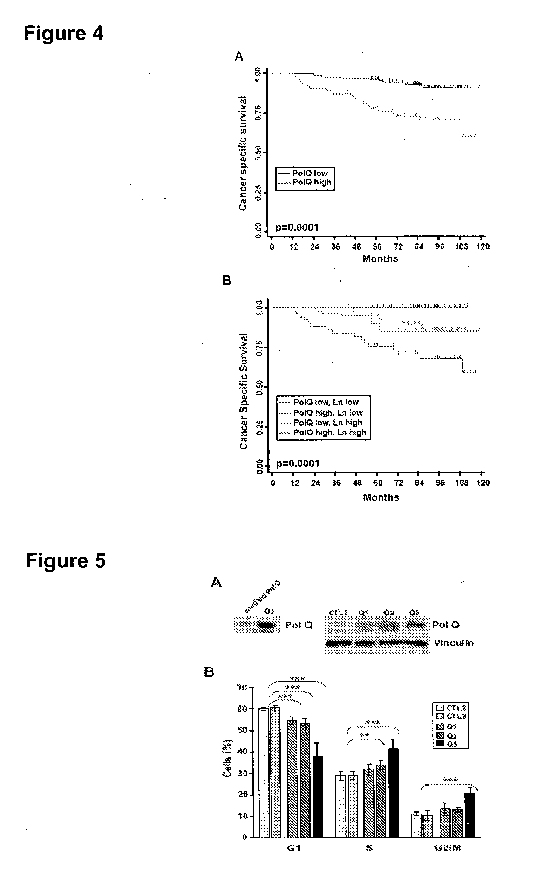

**Figure 4****. Effect of *POLQ* gene expression level on cancer-specific survival of patients.** (A) Kaplan-Meier survival of breast cancer patients, according to level of DNA *POLQ* expression in the primary tumor. Patients from the French cohort (n=203 instead of 206, 3 samples without *POLQ* determination being discarded). p values taken from each log-rank test are indicated. (B) Kaplan-Meier survival of pair wise comparison between *POLQ* gene expression in the primary breast tumor and the number of positive lymph nodes. Patients from the French cohort (n=203)). Log-rank chi-square (X2) and p values are given. Pq stands for *POLQ* and Ln for positive lymph node.
**Figure 5****. Cell homeostasis of POLQ over-expressing clones** (A) Validation of clones stably over-expressing POLQ. Cell extracts (100 µg) from control cells stably transfected with an empty vector (CTL2) as well as from clones stably transfected with *POLQ* cDNA (Q1, Q2 and Q3) were separated on 5% SDS-PAGE, electro-transferred to PVDF membranes and analyzed by immunoblotting with affinity-purified antibodies anti-POLQ (15) and anti-vinculin as an internal control of loading. Purified POLQ (50 ng) is used as a control of size; (B) Consequences on the cell cycle. The number of cells in each phase of the cell cycle was determined by flow cytometry after DNA staining by propidium iodide. At least 3 independent experiments were analyzed. The mean and standard error were determined and p-values were calculated by Student's t-test.

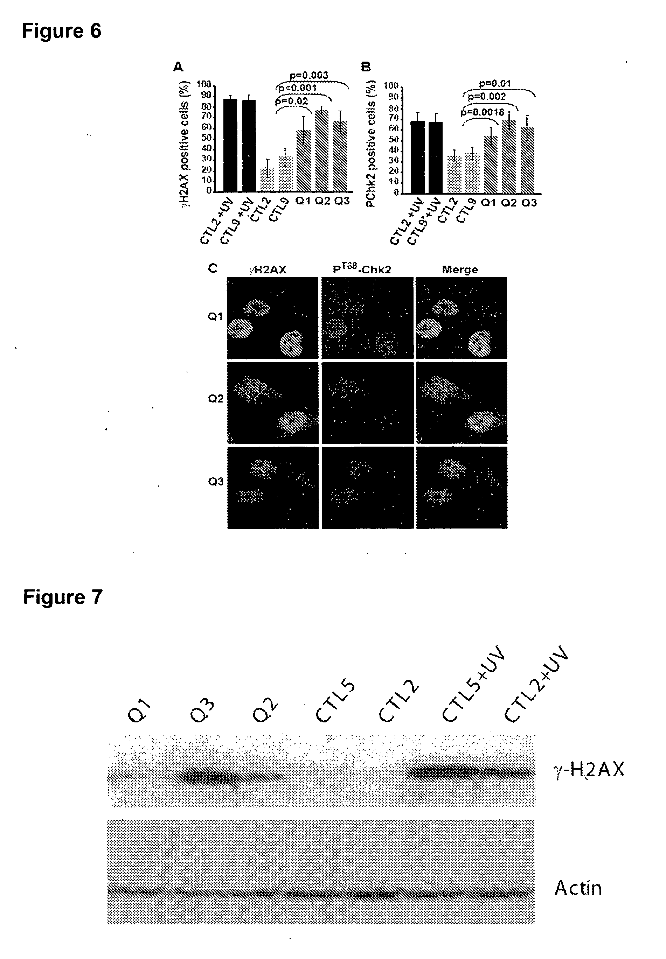

**Figure 6****. ATM-CHK2 DNA damage checkpoint in POLQ over-expressing clones** Quantification of the average number of cells positive to γ-H2AX (A) and PT68-CHK2 (B) staining. Control (CTL) clones treated with UV (10 J.m-2) were used as positive controls. For quantification, a minimum of 100 cells were analyzed in at least 3 independent experiments. The mean and standard error were determined and p-values were calculated by Student's t-test; (C) Representative confocal microscopy images of cells over-expressing POLQ (Q1, Q2, Q3).
**Figure 7****.** γ**-H2AX detection**. 250,000 sub-confluent cells were harvested then sonicated. Total protein extract were resolved by electrophoresis on a 15% SDS polyacrylamide gel. The gel was transferred to a Hybond-P membrane (Amersham, Arlington Heights, IL), which was probed with monoclonal anti -H2AX antibody (Upstate, Lake Placid, NY) overnight at 4°C. Horseradish peroxidase-goat antimouse antibody was used as the secondary antibody (Sigma, St. Louis, MO). The membrane was then incubated with enhanced chemiluminescence reagent (Amersham). Equivalent amounts of loaded extracts were assessed by immunoblotting the membrane with an anti-actin antibody.

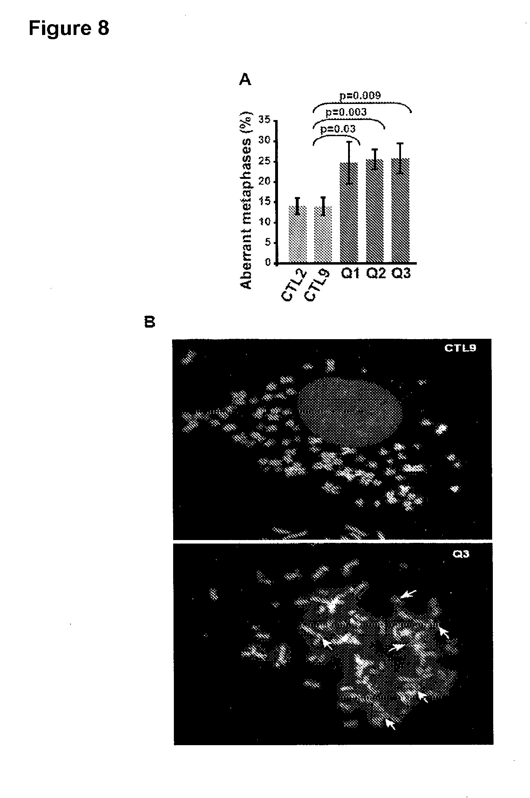

**Figure 8****. DNA breaks and chromosomal abnormalities in POLQ over-expressing clones** (A) Quantification of aberrant metaphases in control clones (CTL2 and CTL9) and in clones over-expressing POLQ (Q1, Q2 and Q3). Cells were collected 3 hours after colcemid treatment and metaphase spreads were prepared. For quantification, a minimum of 100 metaphases were analyzed in 3 independent experiments. The mean and standard error were determined and p-values were calculated by Student's t-test; (B) Representative microscopy images of metaphase spreads from CTL9 and Q3 clones. Arrows indicate chromosomal abnormalities.

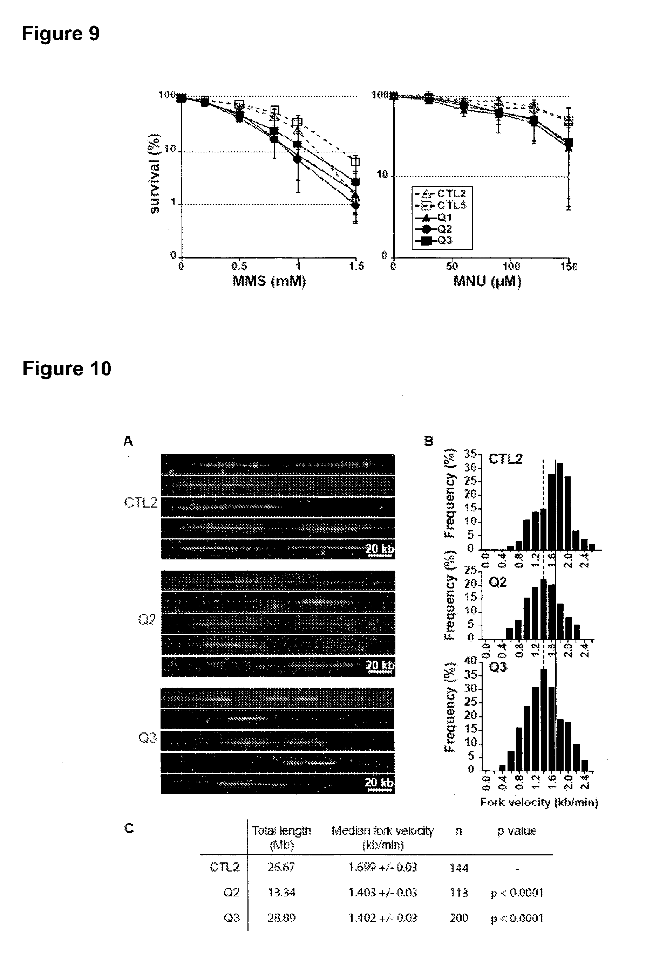

**Figure 9****. Sensitivity to alkylating agents.** Drug sensitivity was determined by clonogenic assay. Sensitivity of POLQ overexpressing (Q1, Q2, Q3) and isogenic control (CTL2, CTL5) cells to methyl methanesulfonate (MMS) and N-methy-N-nitrosourea (MNU). Survival is expressed as the relative plating efficiency of treated cells to the controls. The doses of MMS and MNU are displayed on the x axis in a linear scale, while the fractions of surviving colonies are on the y axis in a logarithmic scale. Results are the mean +/- SD of at least three separate experiments performed in duplicate.
**Figure 10****. DNA replication fork velocity in cells over-expressing POLQ** (A) Representative image of a combed and immunostained DNA fiber in control cells or cells over-expressing POLQ; (B) Histograms showing replication fork velocity distribution in a control clone (CTL2) and in two clones over-expressing POLQ (Q2 and Q3); (C) Total length is the sum in Mb of all DNA fibers that were studied for each clone; "n" is the number of tracks of IdU and CldU scored in each clone. The median value of the population is given in kb/min. The uncertainty of median replication fork velocity is given in units of median absolute deviation. Mann-Whitney test was applied to compare Q2 and Q3 data sets with the control of the experiment, CTL2.

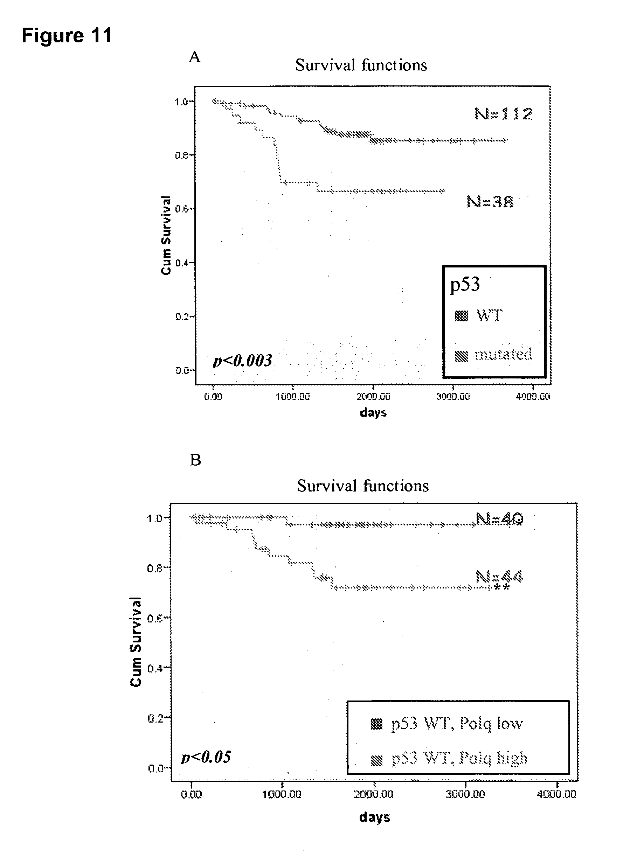

**Figure 11****. Effect of *POLQ* gene expression level on p53-specific survival of patients.** (A) Kaplan-Meier survival of breast cancer patients (n=150), according to the status of the p53 gene (wild-type vs mutated cDNA sequence). To compare these survival curves, the log-rank test was used. This test calculates a p-value testing the null hypothesis that the survival curves are identical in the two populations. p value taken from the log-rank test is indicated. (B) Kaplan-Meier survival of breast cancer patients with p53^{wt} tumors (n=84) according to the level of expression *of POLQ.* To compare these survival curves, the log-rank test was used. This test calculates a p-value testing the null hypothesis that the survival curves are identical in the two populations. The p value is given.

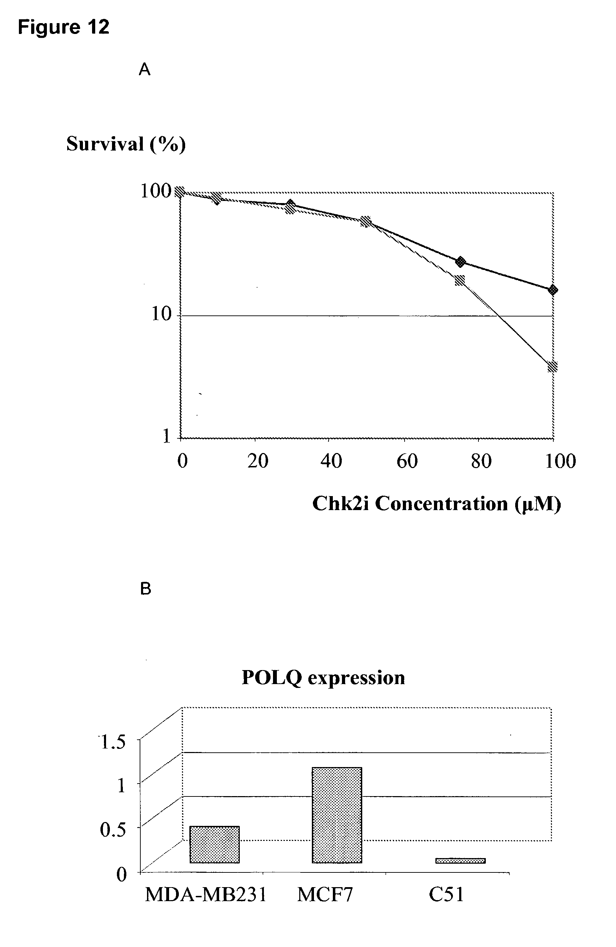

**Figure 12****. Effect of POLQ gene expression level on sensitivity to a Chk2 inhibitor**. (A) MCF7 cells show a higher sensitivity to Chk2 inhibitor II Hydrate (Sigma RefC3742) than MDA-MB231 cells. Survival is determined in a clonogenic assay. MCF7: squares; MDA-MB231: diamonds (B) POLQ expression is higher in MCF7 than in MDA-MB231. Gene expression levels are assayed by RT-PCR. C51 is a normal breast cell line.

### EXAMPLES

### 1. Material and methods

### 1.1. Study design, patients and tumor samples, differential gene expression

Patients included consisted of a subset of patients from an adjuvant multicentric phase III clinical trial (PACS01 trial). The results of this clinical trial have been published elsewhere (Roché et al. J Clin Oncol 24: 5664-5671, 2006). Tumor samples from this cohort (n=206) were divided into two sets for genotyping (n=101 and n=105). Normal breast tissues were obtained at the Claudius Regaud Institute (Toulouse, France) and taken from the surgical specimen, at more than 3 cm from the breast cancer. Characteristics of patients and tumors from the cohort are described in Table 1.

**Table 1: Baseline characteristics of patients and tumors**

| | French cohort | | | |
|---|---|---|---|---|
| | Set 1 (n= 101) | | Set 2 (n = 105) | |
| Characteristics | n. of patients | % | n. of patients | % |
| Age (years) | | | | |
| Median | 49 | | 50.8 | |
| Range | 29-64 | | 31-64 | |
| Premenopausal Surgery | 70 | 69.31 | 58 | 55.24 |
| Local excision | 60 | 59.41 | 54 | 51.43 |
| Mastectomy | 41 | 40.59 | 51 | 48.57 |
| Pathologic tumor size (mm) | | | | |
| Median | 23 | | 25 | |
| Range | 6-70 | | 10-150 | |
| Histological type | | | | |
| Ductal carcinoma | 82 | 81.19 | 80 | 76.19 |
| Lobular carcinoma | 12 | 11.88 | 11 | 10.48 |
| Other | 7 | 6.96 | 14 | 13.33 |
| Grade | | | | |
| I | 22 | 21.78 | 9 | 8.57 |
| II | 34 | 33.66 | 39 | 37.14 |
| III | 42 | 41.58 | 54 | 51.43 |
| Not graded | 2 | 1.98 | 2 | 1.9 |
| Missing | 1 | 0.99 | 1 | 0.95 |
| Number of positive nodes | | | | |
| ≤1 | 43 | 42.57 | 30 | 28.57 |
| >1 | 58 | 57.42 | 75 | 71.42 |
| Estrogen receptors | | | | |
| Positive | 68 | 67.33 | 74 | |
| Negative | 30 | 29.7 | 29 | |
| Missing | 3 | 2.97 | 2 | |
| Progesterone receptors | | | | |
| Positive | 42 | 41.58 | 41 | 39.05 |
| Negative | 56 | 55.45 | 62 | 59.05 |
| Missing | 3 | 2.97 | 2 | 1.9 |
| HER2 | | | | |
| Positive | 16 | 15.84 | 13 | 12.38 |
| Negative | 66 | 65.35 | 87 | 82.86 |
| Missing | 19 | 18.81 | 5 | 4.76 |
| Treatment | | | | |
| FEC (only) | 49 | 48.51 | 57 | 54.29 |
| FEC + TXT (only) | 52 | 51.49 | 48 | 45.71 |
| Other | 0 | 0 | 0 | 0 |
| Tamoxifen | 57 | 56.43 | 67 | 63.8 |

All the tumor tissue samples were surgically collected and immediately snap-frozen in liquid nitrogen and stored until RNA extraction. French tumor RNA was prepared on the same platform (IPSOGEN, Marseille, France) by the CsCl-cushion technique as described (Chirgwin J. et al: Biochemistry 18:5294-5299, 1979). During the extraction process, all tumor samples were controlled for sufficient tumoral cellularity (i.e. >50% tumor cells) by performing Hematoxylin and Eosin staining. RNA extraction from 7 normal breast tissues was performed on thirty 10 µm-thick sections of the frozen tissue. This method allowed morphological control of the tissue processed for RNA extraction. Slides for each sample were stained with haematoxylin-eosin slides and analyzed by a pathologist. Each normal breast sample was reviewed in order to (i) ascertain the absence of any neoplastic lesion; (ii) quantify the percentage of normal epithelial, fibrous and adipous components.. The quality and the quantity of all French RNA samples were assessed using the Agilent BioAnalyzer 2100 and only RNA presenting a suitable ratio 28S/18S (≥ 1.5) was selected for analysis.

For the French cohort, total RNA was reverse transcribed using the High-Capacity cDNA Archive Kit (Applied Biosystems). The level of transcripts was measured in triplicates by using the TaqMan low density array (Applied Biosystems). All studied genes were amplified in triplicate from tumor and normal samples using the TaqMan Universal PCR Master Mix and the TaqMan Low Density Array technology (Applied Biosystems). PCR amplifications were performed with the TaqMan Low Density Array technology and either the 7900HT fast real time PCR system (Toulouse, batch 1). For the French cohort, we normalized gene expression between samples by using two controls (*HMBS, IPO8*), which were amplified from each cDNA. These two control genes were selected by using the Genorm program as the most stable among 16 tested (B2M, TFRC, YWHAZ, RPLO, 18S, GUSB, UBC, TBP, GAPDH, PPIA, POLR2A, ACTB, PGK1, HPRT1, IPO8, HMBS) on the TaqMan Low Density Human Endogenous Control Array (Applied Biosystems). The primer DNA context sequences as well as the Applied Biosystems references used for analysis of each DNA polymerase and control genes are listed (Table 2). To analyze the variation of expression, T/N ratios between normalized values were calculated where T and N correspond respectively to tumor value and median of normal values. Otherwise, for analyses regarding *POLQ* expression and patient survival, *IPO8* was chosen as a normalizer as it was the most stable gene out of the three chosen control genes. Indeed the calculated variances related to set 1/n=101 and set 2/n=105 were the same (0.0033 for set1 and 0.0031 for set 2, the T test p value being of 0.6510, data not shown)..

**Table 2: DNA context sequences and AB references**

| SEQ ID NO. | Assay ID | Context Sequence | Gene Symbol | RefSeq | Localization |
|---|---|---|---|---|---|
| 1 | Hs00183533_m 1 | AGGGGAATTGATCAG TGCATTCCAC | IPO8 | NM_006390.2 | exon 20-21 |
| 2 | Hs00609297_m 1 | GCGGCTGCAACGGCG GAAGAAAACA | HMBS | NM_000190.3 | exon 1-2 |
| 3 | Hs00213524_m 1 | TACAACCAACCAGGTG TGGTATTTC | POLA | NM_016937.1 | exon 30-31 |
| 4 | Hs00172491_m 1 | CTGTTTGAAGCGGGAT GGATGGCAA | POLD1 | NM_002691.1 | exon 1-2 |
| 5 | Hs00173030_m | CTTTGAAGAGGTGTGT GATGAGATT | POLE | NM_006231.2 | exon 16-17 |
| 6 | Hs00249411_m | GGGAAACATGGGGTG GGTATATGGC | REV1L | NM_016316.1 | exon 2-3 |
| 7 | Hs00197814_m 1 | TCACACAATAAGGTCC TGGCAAAAC | POLH | NM_006502.1 | exon 5-6 |
| 8 | Hs00958495_m 1 | GCAGAAAGCGGGCT CCAGCACCAC | POLM | NM_013284.1 | exon 6-7 |
| 9 | Hs00211963_m 1 | GCCACGAAGGGGTCC AGATTTTATG | POLK | NM_016218.1 | exon 2-3 |
| 10 | Hs00161301_m 1 | AAAAGCCCAGGGAGA TTGGTGGACG | REV3L | NM_002912.1 | exon 13-14 |
| 11 | Hs00203191_m | GATTGAGCAGACAGT CCAGAAAGCA | POLL | NM_013274.2 | exon 7-8 |
| 12 | Hs00160263_m | GAGTTAGTGGCATTGG TCCATCTGC | POLB | NM_002690.1 | exon 5-6 |
| 13 | Hs00394916_m 1 | TGGAGCAGGGAAGGA GCGGCTGGCT | POLN | NM_181808.1 | exon 18-19 |
| 14 | Hs00200488_m 1 | CCAGCTCGCAGGGAG TTCATGATCA | POLI | NM_007195.1 | exon 1-2 |
| 15 | Hs00198196_m 1 | GCCTTTCCCAGGTGGT TCAATACTG | POLQ. | NM_006596.3 | exon 26-27 |

### 1.2. Statistical Analysis

In the French cohort, the major parameters of analysis when comparing the expressions in cancer tissues were the individual T/N ratios normalized to expression of the controls *HMBS and IPO8*. Otherwise, in the analyses of *POLQ* expression in relation to survival, T/N ratios for *POLQ* were normalized to *IPO8* only. Correlations between genes were assessed with a non parametric Spearman's correlation (Spearman's rho). Besides these analyses, the expression data were transformed into binomial data (more or less than 1): thus we could use binomial exact tests to evaluate the significance of gene over- and under-expression defined as unbalanced groups around 1. Relative quantities of *POLQ* mRNA levels were transformed into binomial data. In the French cohort all tumors with expression levels below or equal to 0.063 were defined as low expression. This cut off was defined in relation to survival in order to identify two statistically different populations of patients. Said cut off corresponds to a ratio *of POLQ* mRNA levels of 15.8. A low number of metastatic lymph nodes was defined as a lymph node counts equal to 1 or less, distinct from patients with 2 or more metastatic axillary nodes. The cancer specific survival was defined as the interval between the date of breast surgery and date of death or last-follow-up news (censored data). Patients dead from another cause are considered as a censored observation. Survival rates were estimated according to the Kaplan-Meier method and the log rank test was used to assess the differences between the groups. Cox's proportional hazards regression model using a backward selection procedure and likelihood ratio test was used to investigate the impact of prognostic factors on cancer specific survival. All variables associated with p<0.05 on univariate analysis were included in the initial model. All p-values were two sided. For all statistical tests, differences were considered significant at the 5% level.

### 1.3. Subcloning of POLQ

The human *POLQ* cDNA was transferred into the vector pcDNA 3.1 (Invitrogen) in two steps. First the pFast Bac HTC POLQ containing *POLQ* cDNA (Seki et al. Nucleic Acid Res. 31:6117-6126, 2003) was digested with *RsrII* and *XhoI* to give the N-terminal part of *POLQ* cDNA (1.2 kb). This product was subsequently inserted into pcDNA 3.1 (Invitrogen), previously digested with *EcoRV* and *XhoI*. The C-terminal part *of POLQ* cDNA (6.7 kb) was then isolated by digestion of the pFast Bac HTC POLQ with *XhoI* and *SacII* and inserted in the pcDNA 3.1 vector already containing the N-terminal part of *POLQ* cDNA after digestion of this vector with *XhoI.* The entire sequence of the *POLQ* cDNA was then confirmed.

### 1.4. Cell engineering

MRC5-SV cells (ATCC) were grown and transfected as described (Pillaire et al. Cell Cycle 6: 471-7, 2007). POLQ overexpression was immuno-detected as published (Seki et al. Nucleic Acids Res 31: 6117-6126, 2003), using purified protein as a size control.

Cell cycle analysis studies were performed as also published (Bergoglio et al. J Cell Science 115: 4413-4418, 2002). Cell cycle analysis and cytotoxicity studies were performed as previously published (Bergoglio et al. J Cell Science 115: 4413-4418, 2002). Treatment was 1h at 37°C for methyl methanesulfonate (MMS) and continuous for N-methy-N-nitrosourea (MNU). Immunodetection of γ-H2AX and PT68-CHK2 was carried out according to Rao *et al.* (Rao et al. Mol Cancer Res 5:713-24, 2007). For each clone, a minimum of 100 metaphases were analyzed in 3 independent experiments (Bergoglio et al. Cancer Res 62: 3511-3514, 2002).

### 1.5. DNA combing and statistical analysis

Experiments were performed as previously described (Pillaire et al. Cell Cycle 6: 471-477, 2007). Basically, replicated DNA is first labeled with two consecutive pulses of halogenated nucleosides, iododeoxyuridine (IdU) and chlorodeoxyuridine (CldU) for 20 min each, and then isolated and stretched as recently described (Pillaire et al. Cell Cycle 6: 471-477, 2007). Newly replicated regions ("replication tracks") in the stretched DNA molecules can then be visualized and measured by immunostaining with antibodies that are specific to CldU or IdU. In the present case, cells were successively labeled for 20 minutes with 100 µM IdU and CldU (Sigma-Aldrich).

### 1.6. Sequencing of p53 and analysis of mutations

Exons 4 to 9 of p53 were amplified in a semi-nested PCR reaction carried out according to the manufacturer's recommendations: 2 ul of cDNA was added to a mix containing 45 µL of PCR supermix (Invitrogen) and primers Gil (5'TGA TGC TGT CCC CGG ACG ATA TTG AAC3') and Rev10 (5'CTT CCC AGC CTG GGC ATC CTT G3'). 1 ul of this PCR product was then added to a second PCR reaction with primers Gil and Rev9 (5'CTT CTT TGG CTG GGG AGA GGA3'). The amplification reaction consisted of one cycle of 94°C for 3min, followed by 35 cycles of 94°C for 30 sec, 60°C for 45sec and 72°C for 1 min30sec and a final step of 72°C for 8min.

Amplified products were purified from 1% agarose gels with QIAquick Gel Extraction Kit (Qiagen) and submitted for sequencing at Ninewells hospital DNA analysis facility (Abi 3730 Genetic Analyser) using the primers Gil and MP9ER (5'CTC CCA GGA CAG GCA CAA ACA CG3'). Sequences obtained were aligned to p53 sequence NC 000017-9 from GenBank using the program Multalin (Multiple sequence alignment with hierarchical clustering) (Corpet F; Nucleic Acids Res 16(22): 10881-90, 1988). Mutations were confirmed by re-amplifying the sample and re-sequencing. Only exons 4 to 8 were analysed (from nucleotide 11407 to 14067 on sequence NC 000017-9) Mutations were further validated using the Mutation validation tools from the p53 IARC database (http://www-p53.iarc.fr/MutationValidationCriteria.asp) (Petitjean et al. Hum Mutat. 28(6):622-9, 2007) Sequences were then classified according to the type of mutation they contained: WT or Silent (0), Missense (1) and Nonsense (2).

### 2. Results

### 2.1. POLQ is the DNA polymerase gene most up-regulated in breast tumors.

Real-time PCR was performed to generate gene expression profiles from a series of breast carcinomas (n=101, Table 1). Relative expression levels of DNA polymerases for each tumor, normalized to the median expression level of 7 non-tumor breast tissues, are shown in Figure 1. The data clearly indicates that the *POLQ* gene is more highly expressed in tumor tissues of almost all patients compared to normal tissues, probably because the expression in non-tumor tissues is exceptionally low (Table 3). Among all the polymerase genes analyzed, for the replicative (POLA, POLD and POLE) or specialized (POLH, POLL, POLM, POLN, POLK, POLB, POLI, POLZ/REV3L and REV1) enzymes, *POLQ* showed the highest level of relative T/N expression. The expression of *POLQ* was up-regulated by 3 to 26-fold in tumor samples compared to normal tissues. In 70 out of 101 tumors (69.3%) *POLQ* expression was up-regulated by 5-fold or greater.

**Table 3: Ct values in tumor and normal tissues for each DNA polymerase gene, as derived from Q-PCR analysis**

| **SET1** | **PO LA1** | **PO LD1** | **PO LE** | **RE V1** | **PO LH** | **PO LM** | **PO LK** | **RE V3L** | **PO LL** | **PO LB** | **PO LN** | **PO LI** | **PO LQ** | ***IPO* 8** | ***HM BS*** |
|---|---|---|---|---|---|---|---|---|---|---|---|---|---|---|---|
| ***Tumors*** | | | | | | | | | | | | | | | |
| **PACS01 -BEG-BR001** | 26,89 | 26,95 | 26,50 | 26,96 | 26,83 | 27,76 | 25,42 | 26,55 | 27,88 | 27,33 | 30,85 | 28,36 | 28,89 | 25,44 | 27,30 |
| **PACS01 -AVA-BR-003** | 26,9 4 | 27,9 3 | 26,5 6 | 26,7 9 | 27,4 7 | 27,3 2 | 25,9 3 | 26,9 4 | 27,6 0 | 26,4 2 | 29,7 9 | 27,8 5 | 30,8 2 | 25,9 4 | 27,5 3 |
| **PACS01 -AVA-BR004** | 27,0 9 | 27,9 3 | 26,9 5 | 26,4 3 | 26,9 0 | 27,9 3 | 25,6 7 | 26,7 7 | 27,8 5 | 27,8 9 | 29,8 1 | 28,0 1 | 31,7 5 | 25,6 6 | 27,9 4 |
| **PACS01 -AVA-BR-005** | 27,8 7 | 27,9 6 | 26,9 3 | 27,3 0 | 27,8 2 | 27,7 3 | 25,9 9 | 27,4 1 | 27,8 7 | 25,9 3 | 31,5 9 | 28,7 4 | 29,2 1 | 25,9 0 | 26,9 5 |
| **PACTS01 -AVA-BR006** | 27,2 9 | 27,9 1 | 27,4 6 | 26,9 6 | 27,8 2 | 28,0 6 | 26,1 0 | 26,9 3 | 27,9 1 | 27,9 3 | 30,9 9 | 28,7 9 | 30,8 2 | 26,3 3 | 28,6 1 |
| **PACS01 -AVA-BR009** | 27,2 2 | 27,0 7 | 26,9 0 | 27,8 2 | 27,8 9 | 27,9 1 | 26,8 1 | 26,6 3 | 28,4 9 | 27,9 3 | 30,8 4 | 28,9 9 | 29,8 5 | 26,7 1 | 28,3 4 |
| **PACS01 -BEG-BR002** | 27,6 4 | 27,6 3 | 26,9 3 | 26,9 0 | 26,8 8 | 27,9 4 | 25,7 4 | 26,8 3 | 27,7 5 | 27,7 8 | 28,8 7 | 27,9 7 | 32,6 4 | 25,9 2 | 27,8 8 |
| **PACS01 -CJP-BR108** | 27,3 0 | 26,5 9 | 26,7 2 | 27,5 8 | 27,8 8 | 28,0 3 | 26,0 6 | 26,9 4 | 28,0 9 | 27,0 0 | 30,6 6 | 27,9 2 | 29,6 2 | 25,8 1 | 28,5 0 |
| **PACS01 -CJP-BR109** | 26,9 2 | 26,9 2 | 26,9 3 | 26,9 8 | 27,5 8 | 28,2 5 | 25,8 2 | 27,3 2 | 28,3 7 | 27,8 3 | 31,8 7 | 28,8 7 | 29,0 1 | 26,1 1 | 28,9 4 |
| **PACS01 -CJP-BER110** | 26,6 2 | 26,9 5 | 26,4 1 | 26,8 9 | 26,9 6 | 27,9 7 | 25,8 3 | 27,8 8 | 27,1 5 | 27,3 3 | 29,7 1 | 27,1 2 | 30,6 8 | 25,8 8 | 28,6 9 |
| **PACS01 -CJP-BR111** | 26,9 8 | 26,5 5 | 26,9 2 | 27,2 2 | 26,0 5 | 28,7 1 | 26,9 6 | 26,6 9 | 28,7 8 | 27,8 9 | 30,9 5 | 28,9 1 | 30,9 6 | 26,1 5 | 26,8 8 |
| **PACS01 -CJP-BR112** | 27,9 2 | 27,8 5 | 27,4 7 | 27,0 7 | 27,5 9 | 27,9 7 | 25,8 6 | 26,8 1 | 27,9 5 | 27,3 4 | 29,8 7 | 27,9 1 | 32,1 7 | 26,3 0 | 28,4 0 |
| **PACS01 -CJP-BR114** | 27,6 3 | 27,6 8 | 26,9 0 | 26,8 0 | 26,9 1 | 26,8 3 | 25,2 0 | 26,6 6 | 27,5 4 | 27,1 1 | 29,7 9 | 27,9 0 | 31,6 3 | 26.0 5 | 27,9 4 |
| **PACS01 -CJP-BR115** | 26,8 9 | 26,9 7 | 26,4 2 | 26,8 8 | 26,8 9 | 26,9 4 | 25,6 8 | 27,0 1 | 27,9 4 | 26,9 3 | 28,9 7 | 28,0 2 | 30,4 5 | 25,7 8 | 28,0 1 |
| **PACS01 -CJP-BR116** | 27,5 0 | 27,7 5 | 26,8 9 | 26,7 3 | 27,6 1 | 28,3 1 | 25,8 9 | 26,5 8 | 27,5 2 | 27,5 7 | 30,6 3 | 28,0 2 | 31,7 7 | 25,9 2 | 27,9 0 |
| **PACS01 -CJP-BR117** | 26,9 1 | 27,1 2 | 26,2 0 | 26,9 1 | 27,9 0 | 26,8 9 | 25,1 8 | 26,1 8 | 27,5 2 | 27,8 8 | 30,9 6 | 26,9 2 | 31,9 5 | 25,9 3 | 29,1 5 |
| **PACS01 -FBA-BR092** | 27,6 5 | 27,1 2 | 26,8 8 | 26,8 9 | 25,9 2 | 27,9 7 | 26,6 0 | 26,7 5 | 28,4 9 | 27,7 9 | 29,1 5 | 27,0 0 | 30,9 4 | 26,8 2 | 28,8 8 |
| **PACS01 -FBA-BR094** | 26,9 5 | 26,9 6 | 26,6 7 | 26,7 9 | 26,8 9 | 26,9 2 | 25,4 4 | 25,8 5 | 27,9 5 | 29,3 0 | 29,1 2 | 27,7 2 | 30,7 6 | 25,5 8 | 27,9 1 |
| **PACS01 -FBA-BR097** | 27,2 3 | 27,7 2 | 26,9 2 | 26,9 1 | 26,8 6 | 27,3 3 | 25,8 6 | 26,7 8 | 27,8 2 | 28,7 9 | 29,9 6 | 27,9 4 | 30,8 3 | 25,8 6 | 27,8 3 |
| **PACS01 -FBA-BR101** | 26,8 3 | 26,9 3 | 26,5 9 | 26,8 9 | 26,8 5 | 27,8 6 | 26,6 2 | 26,9 2 | 27,8 3 | 27,4 9 | 29,3 2 | 28,2 5 | 29,4 7 | 25,8 3 | 26,8 8 |
| **PACS01 -FBA-BR103** | 27,4 0 | 27,8 4 | 26,9 3 | 27,2 7 | 27,8 3 | 27,6 7 | 26,9 3 | 27,1 4 | 27,8 4 | 28,7 7 | 30,2 0 | 27,9 0 | 30,8 6 | 26,2 8 | 27,8 6 |
| **PACS01 -FBA-BR105** | 26,6 7 | 25,6 0 | 26,2 9 | 26,7 0 | 26,9 1 | 27,0 6 | 24,7 9 | 26,2 3 | 27,3 5 | 27,8 0 | 29,0 3 | 27,8 4 | 29,4 4 | 25,1 6 | 27,8 5 |
| **PACS01 -FBA-BR106** | 27,9 0 | 27,9 5 | 27,9 1 | 26,8 4 | 26,9 3 | 27,4 3 | 25,8 0 | 26,8 2 | 27,9 7 | 29,8 0 | 26,5 9 | 27,8 0 | 31,0 5 | 25,8 7 | 27,9 0 |
| **PACS01** | 27,9 | 27,8 | 26,9 | 26,8 | 27,7 | 27,4 | 25,8 | 26,8 | 27,9 | 26,9 | 28,7 | 27,7 | 33,6 | 26,4 | 28,8 |
| **-HCL-BR119** | 3 | 7 | 1 | 1 | 6 | 8 | 7 | 7 | 4 | 3 | 8 | 3 | 9 | 3 | 4 |
| **PACS01 -HCL-BR122** | 26,9 4 | 25,1 6 | 25,7 8 | 27,8 8 | 27,9 4 | 28,7 0 | 26,5 1 | 26,6 5 | 27,9 7 | 26,8 1 | 30,0 3 | 29,5 9 | 28,8 4 | 25,8 3 | 27,4 2 |
| **PACS01 -HCL-BR123** | 26,6 8 | 27,0 2 | 25,3 0 | 26,2. 7 | 26,8 9 | 27,3 2 | 25,9 0 | 25,7 9 | 27,5 6 | 25,8 9 | 28,8 8 | 27,9 1 | 29,5 1 | 25,3 7 | 28,0 4 |
| **PACS01 -HCL-BR126** | 26,9 4 | 26,8 8 | 25,9 0 | 26,9 1 | 27,8 3 | 27,9 3 | 25,8 9 | 26,8 7 | 27,3 9 | 27,0 3 | 31,2 6 | 28,7 6 | 28,7 9 | 25,9 5 | 27,7 8 |
| **PACS01 -HCL-BR127** | 27,6 3 | 27,7 5 | 26,8 9 | 26,6 4 | 27,3 6 | 27,8 6 | 26,1 5 | 26,8 8 | 27,9 5 | 28,8 4 | 29,8 6 | 28,9 4 | 31,5 0 | 26,8 3 | 28,8 7 |
| **PACS01 -HTE-BR154** | 27,0 1 | 26,9 2 | 25,9 0 | 26,7 4 | 27,5 6 | 26,8 9 | 25,8 0 | 26,5 8 | 27,8 7 | 26,9 2 | 29,3 9 | 27,8 9 | 31,3 0 | 25,7 3 | 28,3 2 |
| **PACS01 -IGR-BR132** | 27,2 1 | 26,9 7 | 26,8 5 | 26,7 8 | 26,9 2 | 27,8 1 | 25,6 5 | 26,8 6 | 27,5 2 | 27,5 6 | 29,0 1 | 27,9 3 | 30,6 9 | 25,9 0 | 28,5 0 |
| **PACTS01 -IGR-BR133** | 26,9 2 | 27,1 8 | 26,5 7 | 26,8 1 | 27,1 8 | 27,9 8 | 25,8 7 | 27,8 3 | 27,9 1 | 28,8 0 | 29,8 9 | 28,9 4 | 29,8 0 | 25,9 4 | 27,8 9 |
| **PACS01 -IGR-BR135** | 25,7 5 | 26,3 6 | 25,4 9 | 26,7 1 | 26,8 8 | 26,9 5 | 24,8 5 | 25,5 9 | 26,9 1 | 25,9 4 | 26,8 6 | 25,9 8 | 29,1 8 | 24,9 6 | 27,0 0 |
| **PACS01 -IGR-BR138** | 25,9 4 | 25,9 6 | 24,9 1 | 26,8 6 | 26,4 7 | 26,8 7 | 25,4 7 | 26,8 8 | 27,5 7 | 27,5 5 | 28,4 1 | 26,9 5 | 27,9 6 | 25,6 5 | 26,9 3 |
| **PACS01 -IGR-BR139** | 27,8 3 | 27,8 3 | 26,9 2 | 26,9 0 | 27,8 8 | 27,6 4 | 25,9 1 | 27,7 8 | 27,7 3 | 26,8 9 | 29,1 3 | 27,6 3 | 31,0 6 | 25,8 7 | 26,9 1 |
| **PACS01 -IGR-BR141** | 27,9 2 | 27,4 0 | 26,9 0 | 26,5 5 | 27,8 0 | 27,9 4 | 26,5 6 | 26,5 5 | 28,8 6 | 28,2 6 | 30,9 3 | 29,8 0 | 29,8 5 | 25,9 4 | 28,5 7 |
| **PACS01 -IGR-BR142** | 27,8 4 | 27,9 6 | 26,9 4 | 27,6 8 | 27,4 7 | 28,0 2 | 26,9 3 | 26,9 4 | 27,9 2 | 27,9 8 | 29,4 7 | 28,3 7 | 33,9 1 | 26,8 1 | 28,8 4 |
| **PACS01 -IGR-BR143** | 26,8 1 | 27,4 4 | 26,3 1 | 26,7 7 | 26,8 8 | 27,4 6 | 25,8 8 | 25,8 7 | 27,4 2 | 27,0 6 | 28,8 9 | 27,8 9 | 30,7 1 | 25,9 3 | 27,6 8 |
| **PACS01 -IGR-BR144** | 27,1 9 | 27,2 5 | 26,9 6 | 26,9 3 | 27,9 0 | 27,8 5 | 26,3 4 | 26,5 8 | 27,9 2 | 27,6 1 | 30,8 4 | 28,9 3 | 31,9 4 | 26,2 3 | 28,8 8 |
| **PACS01 -IGR-BR145** | 26,9 7 | 26,9 9 | 26,8 6 | 26,8 4 | 26,8 4 | 27,7 6 | 25,8 2 | 26,8 8 | 27,4 2 | 27,8 9 | 28,7 3 | 28,0 1 | 29,9 2 | 25,9 0 | 27,6 8 |
| **PACTS01 -IGR-BR146** | 26,9 7 | 26,8 8 | 26,5 9 | 26,8 5 | 26,8 9 | 27,8 6 | 26,0 7 | 26,9 1 | 27,9 0 | 26,9 0 | 30,8 3 | 28,8 0 | 29,6 0 | 26,2 7 | 27,9 3 |
| **PACS01 -IGR-BR147** | 28,2 6 | 27,5 9 | 27,9 5 | 27,8 9 | 28,6 5 | 29,8 4 | 27,4 2 | 28,1 8 | 29,6 6 | 27,6 9 | 32,8 5 | 30,5 6 | 28,8 9 | 27,0 1 | 27,6 5 |
| **PACS01 -IGR-BR148** | 27,6 6 | 28,6 0 | 26,9 6 | 27,8 9 | 27,8 6 | 28,0 6 | 26,6 9 | 27,5 9 | 28,1 0 | 28,9 4 | 30,8 8 | 28,6 3 | 33,0 6 | 26,9 4 | 29,3 9 |
| **PACS01 -IGR-BR149** | 27,8 7 | 27,9 0 | 27,0 3 | 27,9 4 | 27,7 2 | 28,4 0 | 26,9 5 | 27,7 9 | 28,3 9 | 27,9 3 | 30,9 6 | 30,1 1 | 30,1 2 | 26,6 2 | 27,9 4 |
| **PACS01 -IGR-BR150** | 27,9 1 | 27,9 0 | 26,6 3 | 26,9 3 | 27,8 7 | 27,8 1 | 25,9 2 | 26,9 0 | 27,8 9 | 27,9 5 | 29,8 0 | 28,5 0 | 31,2 1 | 26,0 8 | 27,7 7 |
| **PACS01 -IGR-BR151** | 27,8 8 | 27,7 4 | 26,9 2 | 26,8 5 | 27,8 5 | 27,9 1 | 26,1 6 | 27,4 2 | 27,8 0 | 27,2 2 | 30,7 3 | 27,8 2 | 31,8 5 | 25,9 1 | 27,9 3 |
| **PACS01 -IGR-BR152** | 25,4 1 | 26,8 6 | 25,9 5 | 25,9 4 | 25,9 2 | 26,7 0 | 24,6 4 | 25,8 7 | 26,8 3 | 25,8 3 | 26,8 7 | 27,3 1 | 29,8 6 | 24,6 9 | 27,4 5 |
| **PACS01 -IGR-BR153** | 26,9 0 | 27,4 3 | 26,2 0 | 27,4 1 | 27,2 3 | 27,9 0 | 26,8 4 | 27,8 4 | 28,5 6 | 27,6 6 | 29,8 4 | 28,8 9 | 29,9 6 | 25,9 4 | 27,9 6 |
| **PACS01 -JGO-BR011** | 27,9 5 | 27,8 8 | 27,8 4 | 27,9 2 | 27,8 3 | 27,9 4 | 26,8 1 | 27,8 6 | 28,0 1 | 27,8 3 | 30,7 8 | 28,6 1 | 31,1 3 | 26,8 9 | 28,8 9 |
| **PACS01 -JGO-BR015** | 26,9 5 | 26,6 2 | 26,3 9 | 27,2 6 | 28,3 7 | 28,8 5 | 26,9 1 | 26,8 1 | 28,9 4 | 27,8 9 | 28,9 7 | 28,7 8 | 30,1 0 | 26,9 2 | 27,5 6 |
| **PACS01 T-FBE-BR259** | 26,8 4 | 27,4 3 | 26,1 8 | 26,8 3 | 27,6 1 | 27,9 3 | 25,8 8 | 27,7 8 | 27,9 4 | 27,9 4 | 29,7 5 | 28,8 3 | 29,9 9 | 25,8 7 | 28,0 9 |
| **PACS01 T-HCL-BR307** | 27,8 0 | 27,6 1 | 26,7 4 | 26,8 7 | 26,8 - 8 | 27,8 4 | 26,6 7 | 26,9 5 | 28,1 8 | 28,2 6 | 29,4 6 | 27,8 4 | 31,1 2 | 25,8 1 | 28,3 1 |
| **PACS01 T-HCL-BR306** | 27,4 4 | 28,0 8 | 26,8 1 | 27,3 6 | 27,0 1 | 28,0 6 | 26,9 1 | 27,6 9 | 28,5 3 | 27,9 8 | 29,9 3 | 28,9 1 | 30,8 6 | 26,7 8 | 28,8 0 |
| **PACS01 T-HCL-BR309** | 28,7 4 | 27,9 2 | 26,8 0 | 27,5 1 | 27,0 9 | 27,8 6 | 27,9 5 | 27,8 7 | 28,7 0 | 28,7 4 | 30,0 0 | 28,5 5 | 32,5 2 | 26,7 4 | 28,9 1 |
| **PACS01 T-HCL-BR310** | 27,8 4 | 27,9 3 | 26,9 4 | 26,7 9 | 27,2 5 | 27,0 6 | 25,8 1 | 26,4 1 | 27,8 6 | 27,9 0 | 29,9 4 | 27,7 6 | 30,7 5 | 25,7 7 | 28,6 0 |
| **PACS01 T-HCL-BR313** | 27,9 7 | 28,3 5 | 27,5 2 | 27,8 6 | 27,8 5 | 28,0 8 | 26,8 0 | 27,8 5 | 28,8 7 | 28,0 1 | 30,1 8 | 28,8 8 | 31,9 5 | 26,9 3 | 29,0 0 |
| **PACS01 T-HCL-BR314** | 26,9 1 | 27,7 9 | 26,7 9 | 26,9 4 | 26,8 3 | 27,3 1 | 25,2 0 | 26,1 0 | 27,7 8 | 26,9 6 | 28,9 3 | 27,7 5 | 31,7 | 25,9 6 | 27,6 7 |
| **PACS01 T-HCL-BR317** | 27,5 5 | 28,7 6 | 26,6 0 | 27,3 2 | 27,4 0 | 27,8 9 | 25,8 0 | 26,8 8 | 27,9 7 | 28,7 0 | 29,8 0 | 27,9 4 | 32,9 6 | 26,5 6 | 28,8 8 |
| **PACS01 T-HCL-BR318** | 26,9 2 | 26,8 9 | 26,8 5 | 26,9 2 | 26,9 1 | 27,9 2 | 25,9 1 | 26,6 5 | 28,5 1 | 29,7 0 | 29,3 4 | 27,9 3 | 29,9 6 | 25,9 1 | 28,8 6 |
| **PACS01 T-HCL-BR319** | 27,5 6 | 27,6 1 | 27,4 4 | 27,0 9 | 26,6 6 | 27,8 6 | 25,5 5 | 26,8 8 | 28,8 8 | 28,7 7 | 29,2 5 | 27,6 8 | 30,7 3 | 25,9 2 | 27,9 5 |
| **PACS01 T-HTE-BR256** | 26,1 2 | 26,8 6 | 25,3 5 | 26,8 5 | 26,7 2 | 27,4 3 | 25,8 5 | 27,8 2 | 27,8 3 | 26,6 1 | 28,0 8 | 27,6 9 | 28,9 3 | 25,4 1 | 27,8 5 |
| **PACS01 T-IGR-BR260** | 27,3 5 | 28,1 0 | 27,0 0 | 27,1 7 | 27,9 3 | 27,4 8 | 26,7 8 | 27,8 0 | 28,4 1 | 28,5 1 | 30,8 7 | 28,7 6 | 33,7 8 | 26,2 4 | 28,9 3 |
| **PACS01 T-IGR-BR261** | 26,7 3 | 27,3 5 | 25,8 4 | 27,8 6 | 27,2 9 | 27,8 7 | 26,8 0 | 26,5 8 | 28,7 6 | 25,7 4 | 31,8 1 | 27,6 0 | 29,7 0 | 26,9 7 | 27,9 0 |
| **PACS01 T-IGR-BR262** | 26,9 3 | 26,8 6 | 26,5 7 | 27,8 9 | 26,9 2 | 28,1 9 | 27,8 2 | 27,8 7 | 27,9 6 | 27,8 7 | 31,0 2 | 27,8 7 | 30,0 0 | 26,1 9 | 26,9 2 |
| **PACS01 T-IGR-BR265** | 27,6 0 | 27,9 7 | 26,8 8 | 27,2 1 | 27,3 2 | 27,8 7 | 26,3 1 | 26,8 5 | 27,9 3 | 27,9 3 | 29,8 9 | 27,8 2 | 32,2 8 | 25,9 2 | 27,9 7 |
| **PACS01 T-IGR-BR266** | 26,9 2 | 27,3 9 | 26,6 3 | 26,9 2 | 27,9 1 | 27,5 6 | 26,8 1 | 27,2 6 | 27,7 3 | 27,8 1 | 30,7 6 | 28,7 5 | 28,8 5 | 26,6 4 | 27,7 9 |
| **PACS01 T-IGR-BR267** | 27,6 4 | 27,8 4 | 27,6 5 | 27,9 0 | 27,7 6 | 27,9 3 | 26,0 7 | 26,7 1 | 28,4 8 | 26,9 1 | 30,2 8 | 27,3 9 | 31,5 7 | 26,4 8 | 28,4 2 |
| **PACS01 T-IGR-BR268** | 26,9 5 | 27,0 0 | 26,9 4 | 27,9 7 | 27,8 6 | 27,8 8 | 26,2 4 | 27,5 3 | 27,9 2 | 26,9 0 | 30,9 0 | 27,0 2 | 29,9 0 | 25,9 6 | 27,8 7 |
| **PACS01 T-IGR-BR270** | 26,9 0 | 27,6 5 | 26,8 6 | 27,6 4 | 26,6 9 | 27,9 2 | 26,5 5 | 26,7 4 | 28,7 0 | 26,0 0 | 28,6 6 | 27,8 8 | 31,8 9 | 25,8 6 | 27,8 4 |
| **PACS01 T-IGR_B R272** | 27,5 4 | 27,8 9 | 26,8 2 | 27,8 5 | 26,9 4 | 27,7 4 | 26,9 6 | 26,8 8 | 28,9 4 | 27,6 1 | 29,8 | 29,7 8 | | 26,4 5 | 28,8 5 |
| **PACS01 T-IGR-BR273** | 26,7 2 | 27,3 1 | 26,0 4 | 27,8 7 | 26,9 1 | 27,4 1 | 26,2 2 | 27,3 9 | 27,8 9 | 27,9 1 | 29,5 3 | 27,6 3 | 29,8 6 | 25,9 1 | 27,8 9 |
| **PACS01 T-IGR-BR274** | 27,9 6 | 27,8 2 | 26,8 1 | 27,4 9 | 27,2 5 | 27,9 8 | 26,6 8 | 26,8 2 | 28,1 8 | 28,5 7 | 30,8 3 | 28,9 7 | 31,8 7 | 26,2 2 | 28,8 5 |
| **PACS01 T-IGR-BR275** | 26,9 5 | 26,9 0 | 25,9 0 | 27,8 4 | 26,9 1 | 28,0 8 | 26,7 1 | 27,9 1 | 29,8 4 | 27,8 5 | 31,8 7 | 29,2 4 | 28,9 2 | 26,8 4 | 27,1 7 |
| **PACS01 T-IGR-BR277** | 27,1 1 | 27,4 3 | 26,6 5 | 27,6 0 | 27,1 7 | 26,6 0 | 26,8 4 | 27,7 9 | 28,6 0 | 27,8 1 | 30,8 3 | 27,9 7 | 30,8 2 | 25,9 0 | 27,9 1 |
| **PACS01 T-IGR-BR278** | 27,8 7 | 27,8 2 | 27,7 6 | 27,9 2 | 27,6 6 | 28,6 3 | 27,0 7 | 28,6 8 | 27,9 1 | 27,9 0 | 31,8 7 | 28,8 6 | 30,7 4 | 26,5 8 | 27,8 3 |
| **PACS01 T-IGR-BR280** | 27,2 7 | 28,9 4 | 27,0 5 | 27,1 7 | 26,9 2 | 27,8 7 | 25,7 9 | 26,8 5 | 27,8 5 | 27,7 8 | 29,0 2 | 28,8 4 | 30,9 2 | 25,9 4 | 27,9 4 |
| **PACS01 T-IGR-BR282** | 27,8 1 | 27,6 6 | 26,9 9 | 26,9 0 | 27,8 6 | 27,8 6 | 26,3 7 | 26,6 2 | 28,5 8 | 27,9 4 | 29,8 1 | 28,4 4 | 29,8 4 | 25,8 7 | 27,7 2 |
| **PACS01 T-IGR-BR283** | 28,9 4 | 27,9 8 | 27,5 9 | 28,2 6 | 27,9 4 | 28,7 2 | 27,8 5 | 28,8 5 | 28,9 0 | 29,6 5 | 31,0 4 | 28,9 2 | 31,8 4 | 26,8 5 | 28,9 4 |
| **PACS01 T-IGR-BR284** | 27,9 3 | 27,9 2 | 27,0 3 | 27,8 0 | 27,8 6 | 27,8 6 | 25,7 4 | 28,4 6 | 28,9 3 | 28,5 6 | 30,9 4 | 28,7 1 | 30,6 0 | 26,9 3 | 26,9 9 |
| **PACS01 T-IGR-BR286** | 27,5 4 | 28,8 2 | 27,8 9 | 26,6 9 | 27,1 9 | 28,7 6 | 26,8 1 | 27,7 7 | 28,6 7 | 28,9 4 | 26,8 7 | 28,8 9 | 31,9 3 | 26,6 0 | 28,8 8 |
| **PACS01 T-IGR-BR288** | 26,8 1 | 27,0 5 | 26,5 6 | 27,9 5 | 26,9 6 | 26,9 1 | 25,9 0 | 27,9 0 | 27,9 2 | 29,9 5 | 31,9 7 | 27,9 4 | 30,0 9 | 25,9 0 | 27,9 5 |
| **PACS01 T-IGR-BR289** | 26,9 1 | 27,4 1 | 26,9 3 | 27,4 8 | 26,8 0 | 27,0 6 | 25,8 0 | 26,6 6 | 28,5 0 | 29,4 3 | 29,0 8 | 28,7 9 | 30,6 7 | 25,9 4 | 27,9 4 |
| **PACS01 T-IGR-BR290** | 27,4 9 | 27,8 3 | 26,8 6 | 27,8 7 | 27,8 3 | 27,7 4 | 27,4 6 | 26,9 4 | 28,8 6 | 29,0 2 | 30,9 7 | 28,6 3 | 29,7 8 | 25,8 9 | 28,5 4 |
| **PACS01 T-IGR-BR291** | 26,9 6 | 26,7 1 | 26,8 6 | 28,0 2 | 26,8 9 | 28,4 0 | 26,8 8 | 26,8 9 | 29,7 4 | 27,0 6 | 33,0 2 | 28,9 5 | 30,5 4 | 25,8 8 | 26,8 9 |
| **PACS01 T-BEG-BR294** | 27,4 6 | 27,5 0 | 26,3 4 | 27,9 5 | 27,6 2 | 27,8 1 | 26,4 3 | 27,8 7 | 28,4 8 | 27,9 0 | 30,7 3 | 28,8 9 | 31,3 9 | 26,9 1 | 28,6 3 |
| **PACS01 T-IPC-BR-298** | 27,9 6 | 28,1 6 | 26,9 3 | 27,8 2 | 27,9 3 | 27,5 8 | 26,8 3 | 27,8 6 | 28,9 2 | 27,9 2 | 31,9 0 | 28,6 3 | 32,8 8 | 26,8 3 | 28,8 6 |
| **PACS01 T-IPC-BR299** | 26,8 0 | 26,5 0 | 26,2 2 | 27,9 0 | 27,9 1 | 27,8 8 | 26,6 8 | 26,8 1 | 28,1 6 | 29,9 0 | 30,7 2 | 28,8 8 | 29,8 8 | 26,6 3 | 27,0 4 |
| **PACS01 T-IPC-BR300** | 27,2 8 | 27,8 3 | 26,9 1 | 27,7 4 | 27,4 7 | 27,9 4 | 26,8 7 | 26,8 6 | 27,8 8 | 28,3 9 | 29,8 9 | 26,9 6 | 32,5 2 | 25,9 2 | 27,8 2 |
| **PACS01 T-IPC-BR301** | 27,6 7 | 27,4 4 | 27,2 1 | 27,8 1 | 27,7 4 | 27,8 7 | 26,2 0 | 26,7 3 | 28,7 7 | 27,9 0 | 31,0 4 | 29,2 4 | 30,6 2 | 25,8 5 | 27,3 4 |
| **PACS01 T-IPC-BR303** | 26,5 0 | 25,9 9 | 25,2 8 | 26,9 5 | 26,5 9 | 27,7 5 | 25,6 8 | 26,2 5 | 28,8 9 | 26,9 4 | 29,5 5 | 28,8 3 | 27,9 5 | 25,2 6 | 26,7 1 |
| **PACS01 T-IPC-BR304** | 27,8 9 | 28,2 9 | 26,8 8 | 27,4 2 | 26,9 3 | 28,1 3 | 26,4 1 | 26,8 7 | 28,6 1 | 27,4 5 | 29,9 0 | 27,8 4 | 31,9 0 | 25,9 5 | 27,8 6 |
| **PACS01 T-IPC-BR305** | 26,8 8 | 27,1 2 | 25,8 5 | 26,8 9 | 26,9 1 | 27,8 5 | 25,5 9 | 26,4 3 | 27,9 0 | 28,2 7 | 28,3 7 | 27,6 0 | 31,5 3 | 25,9 3 | 28,9 3 |
| **PACS01 T-PPA-BR237** | 26,0 9 | 26,5 7 | 25,8 2 | 26,9 5 | 26,8 8 | 27,8 9 | 25,8 5 | 26,8 5 | 27,8 9 | 26,8 4 | 28,9 5 | 28,5 9 | 28,9 9 | 25,6 5 | 27,9 4 |
| **PACS01 T-PPA-BR241** | 27,1 1 | 27,2 7 | 25,8 5 | 26,8 7 | 27,7 8 | 27,9 4 | 25,8 9 | 26,4 2 | 27,9 1 | 27,9 5 | 29,8 8 | 28,4 3 | 30,0 6 | 26,8 9 | 27,8 6 |
| **PACS01 T-PPA-BR243** | 26,0 2 | 26,9 5 | 25,3 6 | 26,2 6 | 26,9 2 | 26,5 3 | 25,4 2 | 25,9 8 | 27,8 7 | 26,6 7 | 28,3 6 | 27,9 6 | 30,0 0 | 25,8 8 | 28,7 5 |
| **PACS01 T-PPA-BR244** | 27,9 1 | 26,0 6 | 26,8 8 | 26,8 9 | 26,8 9 | 29,1 8 | 26,4 4 | 27,0 3 | 28,9 9 | 27,3 6 | 30,9 9 | 29,8 4 | 28,4 9 | 26,2 3 | 27,9 1 |
| **PACS01 T-PPA-BR246** | 27,8 1 | 27,0 9 | 26,4 4 | 27,1 4 | 27,9 0 | 27,9 3 | 25,9 2 | 27,2 2 | 27,8 7 | 27,9 1 | 29,9 8 | 28,4 3 | 29,8 9 | 25,9 3 | 28,8 0 |
| **PACS01 T-PPA-BR249** | 26,9 2 | 26,3 0 | 25,4 1 | 26,8 9 | 26,7 9 | 26,9 9 | 25,6 5 | 26,7 7 | 27,2 5 | 25,9 2 | 29,6 1 | 27,4 8 | 28,4 3 | 25,4 5 | 27,8 2 |
| **PACS01 T-PPA-BR250** | 27,7 1 | 28,0 6 | 26,9 2 | 27,2 7 | 27,9 5 | 27,9 9 | 26,8 0 | 26,9 2 | 27,9 4 | 28,2 7 | 28,9 3 | 28,0 2 | 31,8 3 | 25,9 4 | 28,8 6 |
| **PACS01 T-PPA-BR251** | 26,2 3 | 26,9 0 | 25,8 6 | 27,5 1 | 26,8 3 | 27,8 9 | 25,9 7 | 26,9 5 | 27,5 3 | 28,8 6 | 31,8 9 | 28,3 2 | 27,4 7 | 24,8 0 | 27,7 8 |
| **PACS01 T-PPA-BR253** | 26,9 0 | 27,5 9 | 26,8 5 | 26,7 4 | 26,9 6 | 27,7 1 | 25,6 2 | 26,7 9 | 27,9 2 | 27,9 5 | 29,8 3 | 27,9 3 | 31,7 7 | 25,2 1 | 28,9 2 |
| **PACS01 T-PPA-BR254** | 25,9 7 | 26,8 9 | 25,8 4 | 26,7 8 | 25,8 9 | 27,9 2 | 25,9 2 | 26,8 5 | 27,8 7 | 26,9 1 | 29,6 2 | 27,9 4 | 28,1 2 | 25,9 6 | 27,9 5 |

| ***Normal tissues*** | | | | | | | | | | | | | | | |
|---|---|---|---|---|---|---|---|---|---|---|---|---|---|---|---|
| **C51** | 26,8 7 | 26,5 6 | 25,2 3 | 27,3 6 | 26,6 1 | 27,0 7 | 25,7 0 | 26,9 8 | 27,7 4 | 27,9 3 | 29,9 3 | 27,8 0 | 29,4 5 | 25,3 2 | 27,2 1 |
| **PORL1** | 29,1 1 | 29,8 8 | 29,7 3 | 28,8 8 | 29,4 8 | 30,4 0 | 26,9 1 | 28,5 6 | 29,8 8 | 28,5 3 | 32,8 8 | 29,8 8 | 33,9 7 | 27,5 7 | 29,7 9 |
| **GARL2** | 29,6 4 | 30,8 6 | 29,7 6 | 28,8 5 | 29,6 7 | 30,3 8 | 27,7 2 | 28,7 5 | 30,0 1 | 28,9 0 | 33,6 0 | 29,3 5 | 35,0 2 | 27,9 3 | 30,3 7 |
| **MISL3** | 28,9 4 | 29,7 5 | 28,8 9 | 27,9 3 | 28,8 8 | 30,0 0 | 26,8 3 | 27,6 9 | 28,9 3 | 27,8 1 | 32,8 9 | 28,8 9 | 32,8 7 | 26,8 4 | 28,8 9 |
| **TROL4** | 28,8 2 | 29,7 6 | 28,5 9 | 28,0 3 | 28,9 1 | 29,6 3 | 26,8 2 | 27,9 0 | 29,8 4 | 27,8 9 | 31,6 7 | 28,5 7 | 33,8 1 | 27,0 2 | 29,3 2 |
| **GEL5** | 28,8 5 | 29,9 4 | 28,9 4 | 27,9 6 | 28,7 3 | 29,7 4 | 27,2 1 | 27,6 9 | 29,5 8 | 28,6 7 | 31,8 8 | 28,8 6 | 33,6 3 | 26,9 9 | 29,1 1 |
| | | | | | | | | | | | | | | | |
| | | | | | | | | | | | | | | | |

| **SET2** | **PO LA1** | **PO LD1** | **PO LE** | **RE V1** | **PO LH** | **PO LM** | **PO LK** | **RE V3L /PO LZ** | **PO LL** | **PO LB** | **PO LN** | **PO LI** | **PO LQ** | **IPO 8** | **HM BS** |
|---|---|---|---|---|---|---|---|---|---|---|---|---|---|---|---|
| ***Tumors*** | | | | | | | | | | | | | | | |
| **PACS01 -AVA-BR007** | 27,8 1 | 27,8 9 | 26,8 9 | 27,8 8 | 27,9 0 | 27,6 8 | 26,5 3 | 27,8 8 | 28,5 1 | 27,8 2 | 28,7 0 | 28,6 6 | 30,9 3 | 26,7 5 | 28,9 3 |
| **PACS01 -AVA-BR008** | 26,8 2 | 27,7 7 | 26,9 3 | 27,3 5 | 27,7 5 | 28,0 1 | 26,9 3 | 28,8 1 | 28,1 7 | 27,4 5 | 30,6 1 | 28,9 5 | 29,7 2 | 26,2 4 | 28,7 2 |
| **PACS01 -FBA-BR085** | 26,6 0 | 26,8 9 | 25,9 3 | 27,0 2 | 26,9 7 | 27,4 4 | 25,8 8 | 26,6 3 | 27,9 2 | 26,8 9 | 29,7 5 | 28,7 9 | 28,7 5 | 25,8 8 | 28,5 4 |
| **PACS01 -FBA-BR087** | 27,8 7 | 27,5 8 | 26,5 1 | 26,9 0 | 27,3 3 | 27,7 7 | 25,7 4 | 26,9 1 | 27,4 8 | 28,3 0 | 29,8 5 | 28,8 3 | 33,0 7 | 26,0 8 | 27,8 8 |
| **PACS01 -FBA-BR089** | 27,9 2 | 28,8 5 | 27,5 0 | 26,7 8 | 27,5 0 | 26,8 7 | 26,8 4 | 26,9 , 6 | 27,8 8 | 27,4 6 | 29,0 4 | 27,9 1 | 33,2 4 | 26,5 1 | 29,3 0 |
| **PACS01 -FBA-BR091** | 27,9 6 | 27,9 0 | 26,8 8 | 26,9 1 | 27,7 7 | 27,2 4 | 26,5 3 | 27,3 7 | 28,1 3 | 25,9 1 | 30,9 3 | 28,4 7 | 29,9 3 | 25,8 7 | 27,8 9 |
| **PACS01 -FLE-BR024** | 27,2 6 | 27,1 9 | 26,0 7 | 26,9 1 | 27,5 4 | 26,9 8 | 25,8 6 | 26,5 8 | 27,8 7 | 26,8 6 | 28,9 0 | 28,3 4 | 31,9 5 | 26,8 2 | 28,8 7 |
| **PACS01 -FLE-BR025** | 26,9 3 | 27,0 0 | 25,9 7 | 26,5 1 | 26,8 8 | 26,8 2 | 25,0 7 | 25,8 3 | 27,9 3 | 27,0 3 | 28,6 0 | 27,9 4 | 30,8 6 | 25,7 5 | 27,3 6 |
| **PACS01 -FLE-BR027** | 26,9 3 | 26,9 4 | 26,5 3 | 25,4 7 | 26,8 1 | 26,9 0 | 25,3 7 | 25,9 0 | 27,8 8 | 26,5 2 | 29,7 9 | 27,8 1 | 29,3 5 | 24,9 9 | 27,5 5 |
| **PACS01 -FLE-BR028** | 27,4 6 | 26,8 9 | 26,7 8 | 25,9 6 | 27,8 9 | 26,4 8 | 25,9 1 | 26,7 2 | 27,9 8 | 27,8 7 | 28,9 1 | 28,5 9 | 29,8 6 | 25,5 5 | 26,9 1 |
| **PACS01 -FLE-BR029** | 26,8 4 | 26,9 3 | 25,8 3 | 26,8 3 | 26,8 5 | 26,8 6 | 25,9 0 | 27,8 9 | 27,8 3 | 27,8 5 | 28,7 8 | 27,5 9 | 29,5 4 | 25,8 2 | 27,5 7 |
| **PACS01 -FLE-BR030** | 26,7 5 | 26,8 4 | 25,4 5 | 26,7 7 | 26,7 8 | 26,7 0 | 25,8 1 | 27,8 7 | 27,2 9 | 26,2 3 | 28,7 8 | 27,8 7 | 29,8 4 | 25,1 9 | 27,9 6 |
| **PACS01 -FLE-BR031** | 27,4 2 | 28,0 3 | 27,4 8 | 26,8 2 | 27,3 7 | 27,7 8 | 25,8 7 | 26,7 3 | 27,7 3 | 27,7 6 | 29,9 6 | 27,8 2 | 31,7 4 | 25,9 3 | 28,8 3 |
| **PACS01 -FLE-BR032** | 26,8 8 | 27,2 0 | 26,9 1 | 26,6 6 | 26,9 4 | 27,8 9 | 25,6 9 | 26,8 5 | 26,9 5 | 26,8 3 | 29,8 2 | 28,9 2 | 29,9 4 | 25,7 8 | 28,5 8 |
| **PACS01 -FLE-BR033** | 27,6 5 | 27,8 4 | 26,7 9 | 26,8 3 | 26,7 2 | 27,0 1 | 25,3 8 | 26,7 7 | 27,8 6 | 27,8 4 | 28,8 7 | 27,7 1 | 29,7 6 | 26,0 4 | 28,6 4 |
| **PACS01 -FLE-BR034** | 27,9 0 | 27,7 4 | 26,7 6 | 26,6 6 | 26,9 2 | 27,5 3 | 25,8 8 | 26,9 1 | 28,2 4 | 28,3 6 | 29,5 7 | 27,9 4 | 30,7 2 | 25,8 6 | 27,9 7 |
| **PACS01 -FLE-BR035** | 26,9 3 | 27,8 2 | 25,8 4 | 26,9 1 | 27,8 0 | 26,3 8 | 25,8 8 | 26,9 1 | 27,8 8 | 27,8 1 | 29,9 8 | 27,5 0 | 31,3 6 | 25,8 0 | 28,6 1 |
| **PACS01 -FLE-BR036** | 26,9 5 | 26,9 4 | 26,1 3 | 26,7 2 | 26,7 0 | 26,9 0 | 25,8 0 | 26,6 4 | 27,8 5 | 26,8 8 | 29,7 1 | 27,9 4 | 30,2 1 | 25,3 6 | 28,8 6 |
| **PACS01 -FLE-BR037** | 25,9 7 | 24,6 8 | 24,9 5 | 26,2 8 | 25,8 1 | 27,7 1 | 25,7 5 | 25,9 1 | 28,0 6 | 26,9 5 | 30,2 0 | 28,1 2 | 27,5 4 | 25,3 0 | 27,4 8 |
| **PACS01 -FLE-BR038** | 26,9 6 | 26,9 2 | 25,3 8 | 26,9 5 | 26,7 8 | 27,0 5 | 25,7 9 | 27,8 0 | 27,5 7 | 27,6 8 | 29,9 2 | 27,9 4 | 29,9 5 | 25,8 0 | 27,8 9 |
| **PACS01 -HBE-BR045** | 26,9 1 | 26,7 7 | 24,9 2 | 26,8 4 | 26,1 0 | 26,8 7 | 25,9 0 | 26,8 4 | 27,8 8 | 26,3 8 | 28,4 0 | 27,3 8 | 27,6 4 | 25,9 4 | 26,8 5 |
| **PACS01 -IPC-BR018** | 27,6 8 | 27,8 2 | 26,8 9 | 26,7 0 | 26,7 9 | 27,8 5 | 25,8 4 | 26,8 6 | 27,8 6 | 28,8 6 | 28,9 1 | 27,7 9 | | 25,9 3 | 28,8 2 |
| **PACS01 -IPC-BR021** | 26,9 7 | 26,9 0 | 26,2 4 | 26,8 3 | 26,8 7 | 27,6 9 | 25,4 4 | 25,9 6 | 27,7 8 | 28,9 3 | 29,9 5 | 27,7 2 | 30,0 0 | 25,4 7 | 27,9 4 |
| **PACS01 -IPC-BR022** | 27,1 9 | 27,9 6 | 26,8 9 | 26,8 5 | 27,5 5 | 27,9 2 | 25,9 5 | 26,4 7 | 27,8 3 | 27,8 4 | 30,8 5 | 27,8 1 | 33,4 0 | 25,9 5 | 28,3 3 |
| **PACS01 -JGO-BR012** | 27,5 0 | 26,8 3 | 25,8 6 | 27,7 . 1 | .26,0 0 | 27,3 6 | 25,8 8 | 26,8 6 | 27,8 5 | 26,8 6 | 29,4 5 | 27,6 6 | 28,9 2 | 26,6 6 | 27,8 8 |
| **PACS01 -JGO-BR014** | 27,9 2 | 27,1 0 | 26,8 2 | 27,9 7 | 25,9 1 | 27,8 8 | 25,9 3 | 26,9 9 | 28,9 2 | 27,6 9 | 30,7 0 | 28,9 2 | 29,8 3 | 25,8 6 | 27,8 0 |
| **PACS01 -JGO-BR016** | 26,9 3 | 27,8 1 | 26,4 0 | 26,7 4 | 26,8 9 | 27,9 1 | 25,4 3 | 26,8 3 | 27,6 9 | 26,4 7 | 29,9 0 | 27,8 9 | 29,9 4 | 25,8 2 | 28,7 1 |
| **PACS01 -OLA-BR041** | 25,9 5 | 26,6 8 | 25,8 3 | 26,9 1 | 25,9 9 | 26,9 0 | 24,3 7 | 26,7 1 | 27,4 3 | 25,4 3 | 28,8 7 | 27,5 0 | 28,5 2 | 25,1 9 | 26,9 1 |
| **PACS01 -OLA-BR042** | 26,9 3 | 26,8 4 | 26,8 1 | 27,6 1 | 26,8 8 | 27,7 8 | 26,0 3 | 27,9 5 | 28,0 4 | 25,9 2 | 30,6 4 | 27,9 5 | 29,9 1 | 25,9 3 | 27,8 6 |
| **PACS01 -OLA-BR043** | 26,9 7 | 27,0 3 | 26,4 0 | 26,9 8 | 26,8 4 | 27,3 0 | 25,7 9 | 26,7 7 | 26,8 6 | 27,4 7 | 29,2 5 | 27,4 9 | 30,9 2 | 25,8 4 | 27,8 9 |
| **PACS01 -OLA-BR044** | 27,9 5 | 27,3 7 | 26,9 2 | 28,2 4 | 27,8 1 | 28,0 7 | 28,9 0 | 27,7 6 | 27,4 8 | 27,7 6 | 30,9 6 | 27,9 0 | 30,7 3 | 26,0 2 | 26,8 7 |
| **PACS01 -PPA-BR047** | 27,1 0 | 25,8 8 | 25,9 3 | 27,5 1 | 27,9 3 | 28,9 7 | 26,8 9 | 26,0 4 | 28,8 3 | 26,8 3 | 31,7 5 | 27,6 4 | 29,0 4 | 25,7 9 | 27,3 0 |
| **PACS01 -PPA-BR048** | 28,0 3 | 27,8 8 | 26,5 0 | 26,9 4 | 27,5 4 | 27,0 4 | 26,3 1 | 26,7 6 | 28,6 7 | 26,9 3 | 31,8 0 | 27,8 9 | 29,3 1 | 26,4 1 | 28,0 8 |
| **PACS01 -PPA-BR049** | 27,3 3 | 27,9 1 | 27,7 1 | 26,8 9 | 27,3 0 | 27,9 1 | 25,9 3 | 26,2 4 | 27,1 6 | 27,8 2 | 29,1 0 | 27,4 9 | 32,4 9 | 25,8 6 | 27,6 9 |
| **PACS01 -PPA-BR050** | 27,0 0 | 27,0 7 | 26,9 1 | 26,7 8 | 25,8 9 | 28,8 3 | 26,6 5 | 26,8 8 | 28,8 6 | 26,1 2 | 32,5 8 | 27,9 5 | 29,8 9 | 26,0 2 | 26,9 0 |
| **PACS01 -PPA-BR053** | 26,9 0 | 27,7 2 | 26,5 7 | 26,7 9 | 26,7 9 | 27,5 9 | 26,8 4 | 26,5 6 | 27,9 0 | 27,9 4 | 30,8 5 | 26,9 5 | 29,8 6 | 26,9 2 | 27,4 6 |
| **PACS01 -PPA-BR057** | 27,7 7 | 27,8 9 | 26,9 4 | 26,9 8 | 27,0 2 | 27,8 7 | 26,8 3 | 26,9 1 | 28,9 0 | 27,1 9 | 29,8 2 | 27,5 9 | 30,9 7 | 26,3 5 | 28,8 9 |
| **PACS01 -PPA-BR058** | 26,9 5 | 26,9 2 | 26,7 2 | 26,8 0 | 27,0 0 | 26,6 7 | 26,2 9 | 26,3 8 | 27,9 0 | 26,8 1 | 29,8 8 | 27,8 2 | 31,0 2 | 25,7 7 | 27,9 9 |
| **PACS01 -RGA-BR061** | 26,7 3 | 26,9 0 | 25,9 5 | 25,8 9 | 26,3 9 | 26,8 6 | 24,9 3 | 25,8 8 | 26,8 7 | 26,9 0 | 28,7 4 | 26,9 2 | 30,9 6 | 24,7 4 | 26,9 5 |
| **PACS01 -RGA-BR062** | 27,0 7 | 27,7 8 | 27,7 3 | 26,9 5 | 26,9 5 | 26,9 0 | 25,9 3 | 26,9 1 | 27,4 6 | 27,6 3 | 29,5 1 | 27,5 7 | 31,1 1 | 25,9 3 | 28,8 0 |
| **PACS01** | 26,9 | 27,8 | 26,9 | 26,7 | 27,1 | 27,0 | 26,3 | 26,4 | 27,8 | 25,9 | 30,9 | 27,9 | 28,8 | 26,1 | 28,9 |
| **-RGA-BR063** | 6 | 7 | 0 | 6 | 0 | 4 | 6 | 6 | 4 | 5 | 2 | 4 | 9 | 7 | 0 |
| **PACS01 -RGA-BR065** | 27,9 1 | 27,9 5 | 26,9 3 | 27,0 2 | 27,6 1 | 27,9 5 | 25,9 5 | 27,7 0 | 27,9 0 | 27,7 4 | 29,2 0 | 28,3 8 | 30,0 7 | 26,1 7 | 28,7 8 |
| **PACS01 -RGA-BR066** | 27,8 9 | 27,6 9 | 26,7 6 | 26,3 2 | 27,8 4 | 27,8 2 | 25,7 2 | 26,9 6 | 27,8 1 | 27,8 2 | 28,9 3 | 27,9 3 | 31,7 7 | 25,8 8 | 27,8 8 |
| **PACS01 -RGA-BR067** | 27,9 9 | 28,9 3 | 27,5 6 | 27,7 9 | 28,8 5 | 27,9 3 | 26,5 5 | 27,8 2 | 28,7 2 | 28,9 4 | 29,8 1 | 29,3 6 | 31,5 4 | 26,7 5 | 29,2 7 |
| **PACS01 -RGA-BR069** | 27,4 1 | 28,8 9 | 26,9 0 | 26,4 4 | 27,7 3 | 26,7 5 | 25,9 1 | 26,8 4 | 27,9 0 | 27,5 8 | 29,9 1 | 28,8 7 | 33,8 9 | 25,9 0 | 28,8 9 |
| **PACS01 -RGA-BR070** | 27,2 4 | 27,2 4 | 26,7 1 | 26,8 9 | 26,8 6 | 26,9 2 | 25,8 7 | 26,9 0 | 27,8 4 | 27,8 8 | 27,5 5 | 27,5 1 | 30,5 0 | 25,9 1 | 27,8 0 |
| **PACS01 -RGA-BR071** | 26,2 2 | 26,3 5 | 25,8 1 | 26,6 4 | 25,9 2 | 26,9 2 | 24,5 3 | 25,7 5 | 26,7 8 | 26,0 7 | 28,3 0 | 26,2 2 | 29,9 5 | 24,9 0 | 26,5 0 |
| **PACS01 -RGA-BR072** | 26,9 1 | 27,0 3 | 26,8 3 | 26,9 1 | 26,7 9 | 27,8 7 | 25,8 9 | 26,9 9 | 26,9 9 | 26,9 0 | 29,0 1 | 26,9 0 | 30,7 9 | 25,5 4 | 26,9 2 |
| **PACS01 -RGA-BR073** | 28,9 6 | 28,7 1 | 28,5 4 | 27,7 0 | 28,5 3 | 28,8 0 | 28,5 9 | 29,9 5 | 28,4 4 | 28,9 3 | 31,6 9 | 29,8 1 | | 27,9 4 | 29,8 6 |
| **PACS01 -RGA-BR074** | 26,8 5 | 27,8 5 | 26,8 4 | 25,9 1 | 26,8 3 | 27,4 4 | 25,9 0 | 27,3 5 | 26,8 6 | 26,6 1 | 29,0 1 | 27,9 6 | 32,0 5 | 25,6 2 | 27,8 7 |
| **PACS01 -RGA-BR075** | 26,6 6 | 26,9 3 | 25,6 1 | 25,7 4 | 26,4 0 | 26,2 6 | 24,9 3 | 25,9 3 | 26,9 6 | 26,9 6 | 28,4 4 | 27,6 4 | 28,8 5 | 25,6 6 | 27,5 3 |
| **PACS01 -RGA-BR076** | 26,8 2 | 27,7 7 | 26,8 6 | 27,8 1 | 26,8 5 | 27,8 7 | 26,6 2 | 27,9 3 | 27,9 4 | 28,8 9 | 30,7 3 | 28,5 5 | 29,8 5 | 26,4 0 | 27,4 4 |
| **PACS01 -RGA-BR077** | 26,9 5 | 27,9 3 | 26,2 2 | 26,8 5 | 27,0 1 | 27,8 3 | 25,9 1 | 26,6 7 | 27,2 0 | 27,5 4 | 29,3 0 | 26,9 8 | 32,5 0 | 25,9 6 | 28,0 6 |
| **PACS01 -RGA-BR078** | 26,9 7 | 26,9 4 | 27,8 0 | 28,1 7 | 26,9 2 | 28,8 0 | 26,6 2 | 27,9 2 | 29,0 8 | 27,5 1 | 31,9 3 | 29,3 3 | 29,6 2 | 26,9 2 | 27,8 2 |
| **PACS01 -RGA-BR079** | 27,5 6 | 27,8 0 | 26,8 9 | 27,0 7 | 27,6 6 | 27,9 5 | 26,0 7 | 27,4 7 | 27,5 6 | 26,9 5 | 29,6 7 | 27,9 1 | 31,1 9 | 26,7 7 | 28,6 4 |
| **PACS01 -RGA-BR080** | 26,7 5 | 26,9 5 | 25,7 9 | 26,6 4 | 26,5 4 | 26,6 9 | 24,8 0 | 26,7 2 | 27,1 7 | 27,0 0 | 28,8 9 | 26,9 6 | 30,4 5 | 25,9 3 | 28,7 7 |
| **PACS01 -RGA-BR081** | 26,9 0 | 26,3 2 | 25,6 3 | 26,9 4 | 25,8 7 | 26,9 2 | 25,0 2 | 26,8 9 | 27,8 8 | 27,7 8 | 29,5 3 | 28,7 3 | 29,0 6 | 25,8 2 | 27,9 1 |
| **PACS01 T-AVA-BR167** | 27,9 5 | 27,6 4 | 26,9 0 | 26,9 7 | 26,9 3 | 27,1 2 | 25,5 2 | 26,6 4 | 27,8 7 | 27,9 6 | 28,6 1 | 27,9 8 | 33,0 0 | 25,7 6 | 28,7 0 |
| **PACS01 T-AVA-BR168** | 27,5 7 | 27,0 4 | 26,9 7 | 27,2 6 | 27,0 2 | 27,6 0 | 25,9 3 | 27,9 2 | 28,6 4 | 26,9 1 | 30,8 4 | 27,9 3 | 30,9 6 | 25,9 2 | 27,8 2 |
| **PACS01 T-AVA-BR170** | 27,6 2 | 26,9 6 | 26,5 6 | 26,8 5 | 27,5 7 | 27,8 9 | 25,9 5 | 26,8 8 | 27,9 5 | 27,9 1 | 29,8 0 | 27,6 9 | 30,3 2 | 26,9 0 | 28,8 0 |
| **PACS01 T-AVA-BR172** | 29,9 0 | 28,3 9 | 28,8 0 | 28,2 4 | 28,8 4 | 28,8 7 | 27,4 1 | 28,7 8 | 28,6 4 | 28,8 2 | 32,8 9 | 28,8 5 | 33,0 8 | 27,9 0 | 28,8 9 |
| **PACS01 T-AVA-BR173** | 27,5 0 | 26,8 7 | 26,0 2 | 27,6 1 | 26,9 1 | 27,9 3 | 26,3 4 | 27,8 8 | 28,4 0 | 27,8 4 | 29,9 2 | 28,9 0 | 29,2 6 | 25,8 6 | 28,4 7 |
| **PACS01 T-CJP-BR156** | 27,6 1 | 27,9 6 | 26,3 1 | 26,6 9 | 26,8 7 | 27,5 8 | 26,3 6 | 26,8 2 | 27,7 6 | 27,8 8 | 27,9 3 | 27,9 0 | 30,7 8 | 26,4 6 | 29,0 4 |
| **PACS01 T-CJP-BR159** | 27,6 3 | 27,4 0 | 26,8 5 | 26,9 1 | 27,0 3 | 27,9 2 | 26,2 3 | 26,9 3 | 28,3 3 | 27,9 2 | 29,8 9 | 28,6 5 | 30,8 1 | 26,0 6 | 28,6 4 |
| **PACS01 T-CJP-BR160** | 27,9 1 | 27,8 4 | 26,7 8 | 26,8 0 | 26,7 8 | 27,7 3 | 25,9 2 | 26,4 4 | 27,8 2 | 27,7 2 | 29,8 0 | 27,9 5 | 32,6 7 | 25,9 0 | 28,9 0 |
| **PACS01 T-CJP-BR164** | 27,4 3 | 27,6 9 | 26,9 4 | 27,2 3 | 27,8 6 | 27,8 7 | 26,8 5 | 27,9 1 | 28,1 5 | 27,9 9 | 30,8 8 | 28,7 3 | 30,2 8 | 26,7 4 | 28,6 7 |
| **PACS01 T-CJP-BR166** | 27,9 9 | 27,9 1 | 26,4 6 | 26,9 9 | 27,5 7 | 27,1 8 | 26,8 0 | 27,8 7 | 27,9 5 | 27,7 5 | 28,7 8 | 27,9 7 | 32,9 7 | 25,9 5 | 27,9 0 |
| **PACS01 T-FBA-BR187** | 26,9 2 | 27,3 8 | 25,9 2 | 26,8 5 | 26,6 4 | 27,2 9 | 25,7 9 | 26,7 6 | 27,3 0 | 25,7 5 | 28,5 5 | 26,9 9 | 29,9 2 | 25,7 7 | 28,2 3 |
| **PACS01 T-FBA-BR188** | 27,8 1 | 27,9 5 | 26,7 9 | 27,4 9 | 27,8 1 | 27,8 9 | 26,7 3 | 27,9 4 | 27,8 9 | 27,9 0 | 29,7 5 | 28,1 5 | 33,8 5 | 26,9 4 | 28,9 6 |
| **PACS01 T-FBA-BR189** | 27,9 8 | 27,6 3 | 26,7 6 | 26,9 7 | 27,7 5 | 27,2 6 | 26,8 0 | 27,8 3 | 28,8 6 | 27,9 4 | 30,9 9 | 28,6 6 | 30,7 1 | 25,9 8 | 27,1 1 |
| **PACS01 T-FBA-BR190** | 28,6 0 | 27,8 0 | 27,8 8 | 27,4 2 | 27,8 4 | 27,9 6 | 26,8 2 | 27,8 5 | 28,8 8 | 27,5 3 | 30,7 4 | 27,9 6 | 32,8 8 | 26,4 3 | 27,9 9 |
| **PACS01 T-FBA-BR192** | 27,3 4 | 27,5 5 | 25,8 8 | 26,8 8 | 26,9 3 | 27,2 9 | 25,6 4 | 27,9 2 | 27,5 1 | 28,9 2 | 29,4 5 | 27,3 0 | 31,1 9 | 25,9 4 | 28,7 3 |
| **PACS01 T-FBA-BR193** | 26,9 1 | 26,9 5 | 25,9 0 | 26,3 4 | 26,4 0 | 27,0 5 | 25,8 5 | 26,8 8 | 26,9 1 | 26,5 7 | 28,9 1 | 27,9 3 | 28,8 8 | 25,5 4 | 26,9 1 |
| **PACS01 T-FBA-BR194** | 27,4 : 2 | 27,6 : 0 | 26,8 5 | 27,3 8 | 26,9 6 | 26,8 7 | 25,8 7 | 28,6 | 27,3 7 | 28,7 8 | 31,0 9 | 26,0 9 | 31,2 3 | 25,9 4 | 26,9 1 |
| **PACS01 T-FBA-BR195** | 24,9 4 | 26,9 0 | 25,7 4 | 24,9 2 | 25,5 9 | 25,8 4 | 24,3 9 | 25,9 3 | 26,0 1 | 26,8 3 | 29,1 7 | 27,0 1 | 29,7 1 | 23,9 1 | 27,2 2 |
| **PACS01 T-FBA-BR197** | 27,8 8 | 27,7 5 | 26,9 0 | 26,8 5 | 27,1 3 | 28,0 4 | 26,5 9 | 26,9 0 | 28,6 6 | 27,7 6 | 29,9 8 | 28,8 8 | 32,0 3 | 26,8 4 | 28,9 1 |
| **PACS01 T-FBA-BR198** | 27,5 2 | 26,9 1 | 26,7 2 | 27,2 5 | 26,7 7 | 27,4 0 | 26,9 2 | 27,1 4 | 27,6 0 | 27,7 0 | 30,2 8 | 26,9 2 | 30,8 5 | 25,9 5 | 26,2 9 |
| **PACS01 T-FBA-BR199** | 26,8 9 | 27,1 0 | 26,8 1 | 27,0 8 | 27,5 1 | 27,3 5 | 25,8 8 | 26,9 2 | 28,6 2 | 25,0 5 | 28,8 0 | 27,6 6 | 31,7 6 | 26,5 5 | 28,4 0 |
| **PACS01 T-FBA-BR200** | 28,9 7 | 27,8 6 | 26,9 0 | 27,9 9 | 27,8 6 | 28,8 0 | 28,0 0 | 28,5 4 | 29,7 9 | 28,8 5 | 31,8 4 | 28,8 9 | 30,9 2 | 26,8 7 | 27,9 2 |
| **PACS01 T-FBA-BR201** | 27,3 2 | 27,8 5 | 25,8 9 | 27,1 6 | 27,7 5 | 26,9 7 | 26,4 0 | 26,9 1 | 27,8 1 | 27,8 9 | 28,9 7 | 27,9 2 | 29,9 6 | 25,9 4 | 27,2 7 |
| **PACS01 T-FBA-BR202** | 26,9 1 | 26,6 9 | 25,6 4 | 26,1 6 | 26,9 2 | 26,2 6 | 25,8 7 | 25,9 2 | 27,9 2 | 28,2 1 | 28,9 4 | 27,8 1 | 29,6 9 | 25,3 1 | 26,8 7 |
| **PACS01 T-FBA-BR203** | 27,9 2 | 27,9 1 | 26,1 4 | 27,5 3 | 26,4 1 | 27,8 8 | 26,8 1 | 27,6 4 | 27,8 3 | 26,9 6 | 30,7 3 | 27,9 6 | 30,9 6 | 26,9 5 | 27,9 3 |
| **PACS01 T-FBA-BR204** | 27,7 8 | 26,5 2 | 25,7 8 | 27,9 3 | 26,8 3 | 27,8 7 | 26,6 0 | 26,9 3 | 27,9 4 | 26,9 3 | 31,2 4 | 28,1 5 | 30,5 7 | 24,9 4 | 26,1 2 |
| **PACS01 T-FBA-BR205** | 27,5 3 | 26,2 7 | 26,6 6 | 27,7 3 | 26,7 0 | 27,9 2 | 26,8 0 | 27,3 9 | 28,7 0 | 26,8 9 | 29,5 8 | 28,1 5 | 28,5 2 | 26,9 2 | 27,6 0 |
| **PACS01 T-FBA-BR206** | 27,8 7 | 27,9 1 | 26,8 9 | 27,9 7 | 27,8 0 | 27,8 1 | 27,5 6 | 27,9 0 | 28,9 3 | 28,7 8 | 30,9 3 | 29,9 0 | 33,0 1 | 27,6 1 | 28,7 8 |
| **PACS01 T-FBA-BR207** | 27,8 1 | 27,3 2 | 27,7 5 | 27,7 7 | 27,8 5 | 27,5 5 | 26,3 3 | 27,8 8 | 27,6 2 | 27,0 0 | 31,0 0 | 27,0 0 | 31,1 0 | 25,8 8 | 28,5 6 |
| **PACS01 T-FBA-BR208** | 26,9 3 | 26,8 8 | 26,6 6 | 26,8 8 | 26,6 2 | 26,9 2 | 25,8 0 | 26,8 5 | 27,9 2 | 25,8 8 | 30,8 3 | 27,9 7 | 29,8 7 | 25,5 5 | 27,8 7 |
| **PACS01** | 27,9 | 27,8 | 27,3 | 27,9 | 27,5 | 28,7 | 26,9 | 27,5 | 28,9 | 28,8 | 30,8 | 29,6 | 30,8 | 26,4 | 28,9 |
| **T-FLE-BR174** | 5 | 9 | 8 | 1 | 2 | 3 | 3 | 3 | 2 | 4 | 8 | 7 | 7 | 4 | 0 |
| **PACS01 T-FLE-BR179** | 27,8 9 | 27,6 | 27,8 0 | 27,8 1 | 27,9 2 | 27,7 5 | 26,9 6 | 26,8 4 | 28,7 6 | 27,3 9 | 30,2 8 | 28,1 9 | 31,4 5 | 26,6 3 | 28,7 4 |
| **PACS01 T-FLE-BR180** | 28,0 4 | 28,0 4 | 27,7 2 | 27,4 0 | 27,7 9 | 28,8 3 | 26,5 6 | 27,9 6 | 28,9 1 | 27,8 8 | 30,0 0 | 28,8 3 | 32,8 5 | 26,9 0 | 28,9 0 |
| **PACS01 T-FLE-BR181** | 27,2 1 | 27,7 7 | 26,9 9 | 26,8 9 | 26,9 0 | 27,5 9 | 25,9 4 | 27,5 4 | 27,8 6 | 27,8 9 | 30,3 1 | 27,4 3 | 30,5 0 | 26,8 6 | 28,5 4 |
| **PACS01 T-FLE-BR184** | 26,8 8 | 27,8 6 | 25,7 8 | 26,5 1 | 26,7 4 | 26,9 6 | 25,6 9 | 26,9 4 | 27,2 2 | 27,9 4 | 28,9 1 | 27,9 0 | 32,8 7 | 26,6 0 | 28,8 8 |
| **PACS01 T-FLE-BR185** | 26,6 4 | 26,6 4 | 26,8 9 | 26,9 3 | 27,9 1 | 24,9 5 | 26,8 9 | 27,0 0 | 27,9 3 | 26,8 8 | 28,7 4 | 26,9 7 | 30,0 2 | 25,8 6 | 29,1 5 |
| **PACS01 T-FLE-BR186** | 27,9 3 | 27,8 1 | 26,8 4 | 27,1 6 | 27,8 5 | 27,8 9 | 26,6 9 | 27,3 7 | 28,6 9 | 27,7 6 | 30,8 2 | 28,9 7 | 30,7 2 | 26,2 0 | 27,9 8 |
| **PACS01 T-JGO-BR231** | 26,5 6 | 26,8 9 | 26,4 2 | 26,9 4 | 26,3 8 | 26,9 5 | 25,8 0 | 26,9 0 | 27,9 0 | 27,5 7 | 28,3 9 | 27,5 7 | 29,8 5 | 25,8 9 | 27,1 5 |
| **PACS01 T-JGO-BR234** | 26,9 2 | 27,7 3 | 25,8 8 | 26,8 8 | 25,8 8 | 26,4 9 | 25,8 9 | 26,8 7 | 27,8 7 | 26,9 0 | 27,8 1 | 27,9 3 | 30,8 5 | 25,9 3 | 28,0 2 |
| **PACS01 T-PPA-BR235** | 26,9 4 | 26,9 6 | 25,9 4 | 26,8 9 | 26,4 9 | 27,0 8 | 25,9 1 | 26,9 1 | 27,5 2 | 28,4 1 | 29,3 7 | 27,9 0 | 29,6 4 | 25,8 3 | 28,5 1 |
| **PACS01 T-PPA-BR236** | 26,6 6 | 26,8 2 | 25,2 8 | 26,8 6 | 26,8 1 | 27,8 5 | 26,8 4 | 27,4 2 | 27,9 6 | 26,9 2 | 30,8 6 | 28,4 4 | 28,8 6 | 25,9 4 | 27,9 1 |
| **PACS01 T-RGA-BR209** | 27,9 5 | 27,3 3 | 26,7 1 | 26,9 7 | 26,8 5 | 26,9 7 | 25,7 5 | 27,7 0 | 27,5 3 | 27,5 6 | 30,1 3 | 27,0 9 | 32,7 9 | 25,8 9 | 27,9 5 |
| **PACS01 T-RGA-BR211** | 28,8 4 | 27,9 8 | 27,8 9 | 27,7 2 | 27,7 9 | 28,2 3 | 26,9 4 | 27,8 5 | 28,9 6 | 29,8 6 | 29,8 2 | 28,9 1 | 31,9 6 | 26,7 2 | 28,8 5 |
| **PACS01 T-RGA-BR212** | 25,8 0 | 25,1 2 | 24,8 0 | 25,7 2 | 27,1 3 | 26,8 3 | 25,8 1 | 25,5 1 | 27,1 5 | 26,9 9 | 27,6 8 | 27,8 1 | 27,8 8 | 25,6 1 | 26,4 4 |
| **PACS01 T-RGA-BR214** | 27,9 3 | 26,9 3 | 26,6 0 | 26,6 4 | 26,8 8 | 27,0 5 | 25,9 2 | 26,9 4 | 27,8 9 | 27,4 7 | 30,9 0 | 27,8 6 | 32,6 3 | 25,9 3 | 27,9 1 |
| **PACS01 T-RGA-BR215** | 27,8 1 | 26,9 9 | 26,9 1 | 27,9 1 | 27,7 1 | 27,9 3 | 26,8 4 | 27,8 8 | 28,8 0 | 27,6 0 | 29,7 3 | 28,8 2 | 30,7 9 | 26,9 5 | 27,6 8 |
| **PACS01 T-RGA-BR219** | 27,6 5 | 27,7 6 | 26,8 5 | 27,4 7 | 27,2 7 | 27,9 0 | 26,8 4 | 27,2 5 | 28,7 6 | 27,9 4 | 29,8 6 | 28,8 5 | 30,9 7 | 26,5 2 | 28,2 6 |
| **PACS01 T-RGA-BR220** | 26,9 5 | 27,0 0 | 26,7 4 | 26,9 6 | 26,8 7 | 26,8 9 | 25,7 9 | 26,9 2 | 27,8 7 | 27,3 4 | 29,8 6 | 27,6 0 | 30,9 8 | 25,8 2 | 27,8 6 |

| ***Normal tissues*** | | | | | | | | | | | | | | | |
|---|---|---|---|---|---|---|---|---|---|---|---|---|---|---|---|
| **C51** | 26,8 9 | 27,6 5 | 26,9 2 | 25,8 0 | 27,9 3 | 27,9 6 | 24,7 4 | 25,9 1 | 27,2 5 | 27,7 6 | 29,0 4 | 27,9 2 | 32,8 3 | 25,9 2 | 27,7 8 |
| **PORL1** | 29,1 1 | 29,5 7 | 29,3 5 | 27,7 8 | 28,8 3 | 29,8 2 | 27,2 4 | 28,1 4 | 29,2 0 | 28,8 0 | 32,4 5 | 29,1 4 | 33,2 4 | 27,3 1 | 28,8 9 |
| **GAR2** | 29,7 5 | 30,8 7 | 29,9 4 | 28,3 7 | 29,6 3 | 30,2 8 | 27,7 8 | 28,6 9 | 29,7 5 | 29,9 1 | 32,8 4 | 29,7 8 | 33,8 7 | 28,0 8 | 30,7 7 |
| **MISL3** | 28,9 2 | 28,9 2 | 28,7 8 | 26,9 4 | 28,7 8 | 29,3 2 | 26,4 4 | 27,9 0 | 28,9 5 | 27,8 4 | 31,7 5 | 28,6 6 | 33,8 9 | 26,9 1 | 28,9 1 |
| **FAV6** | 29,8 8 | 30,8 9 | 29,8 2 | 28,8 7 | 29,4 8 | 30,5 9 | 27,9 4 | 29,0 0 | 29,9 3 | 28,8 9 | 32,5 5 | 29,8 1 | 35,9 4 | 27,9 2 | 29,8 6 |
| **ROV3** | 29,7 4 | 29,4 6 | 29,7 7 | 27,8 7 | 29,7 4 | 29,9 8 | 26,9 4 | 28,8 4 | 29,8 5 | 28,6 2 | 32,1 1 | 28,8 8 | 34,7 1 | 27,8 9 | 29,8 8 |
| **ZIT5** | 29,3 1 | 29,7 5 | 29,4 6 | 28,7 7 | 28,8 8 | 29,6 7 | 27,6 1 | 28,9 2 | 29,8 8 | 27,9 9 | 31,9 5 | 28,7 8 | | 27,6 7 | 29,6 4 |

Interestingly, the most recently discovered DNA polymerase *POLN,* which is the most closely related to *POLQ* (Marini et al. J Biol Chem 278: 32014-32019, 2003), was also significantly over-expressed in tumors. We also noticed a significant under-expression of tumor transcripts for the specialized DNA polymerase *POLB* gene (Fig. 1). Very similar data were found when an independent genotyping analysis (i.e. using an independent batch of Low Density Arrays) was performed in a second set of tumors (n=105, Table 1 and Fig. 2), except that in addition to *POLB, REV1* and *POLK* T/N ratios were mostly lower than 1 in this series. By using a non parametric Spearman's correlation graphical display, we found that in the first tumor set (n=101), expression defects of genes encoding the specialized polymerases POLN, POLM, POLK, POLL and POLI were significantly correlated (Fig. 3), all Spearman > 0.5). In contrast, the specific expression profile that we found for *POLQ* was not clustered in this pattern, showing that *POLQ* gene upregulation in tumors was independent from the regulation of other specialized polymerases.

### 2.2. Intra-tumoral up-regulation of POLQ is associated with a poor clinical outcome

To investigate whether the expression levels of DNA polymerases in tumors could be related to patient survival, a log-rank test was carried out (Fig. 4). For the French cohort, high expression of the *POLQ* gene was significantly associated with poor survival (p=0.0001) (Fig. 4A), strongly suggesting that *POLQ* gene up-regulation is associated with worse survival in breast cancer patients.

Lymph node metastasis is associated with poor survival in breast cancer, as confirmed in both our cohorts (data not shown). Results from the French cohort (Fig. 4B) indicate that patients with high number of positive lymph nodes (2 or more nodes involved with metastatic breast cancer) but low *POLQ* expression had a significantly better survival rate than patients with a high expression of *POLQ* and a high number of lymph nodes (pair wise comparison, French cohort, p=0.0001). It should be highlighted that patients with a high number of lymph nodes and a low level of *POLQ* expression had a survival rate comparable to patients with low number of lymph nodes and high level of *POLQ* expression (pair wise comparison, French cohort, p=0.793). Only the group of patients with a high number of positive lymph nodes and a high level of *POLQ* expression had a poor survival.

A significant statistical association was shown between *POLQ* expression and the number of positive lymph nodes (p=0.0172), histological grade III (p<0.0001), tumor size (Spearman 0.1611, p=0.0237), estrogen receptor (ER) status (p<0.0001), progesterone receptor (PgR) status (p=0.0034), Ki67 expression (p<0.0001) and HER2 status (p=0.0032) (Table 4). Interestingly, the patients displaying "triple-negative" tumors (i.e., ER negative, PgR negative, HER2 negative) showed higher levels of *POLQ* expression (p=0.0422). A multivariate analysis was conducted including the number of lymph nodes involved, histological grade, hormone receptors, vascular invasion and *POLQ* expression. (Table 5). Very interestingly the final models contain lymph node involvement (HR=2.78, CI95%=[1.29;6.01]) and POLQ expression (HR=3.33, Ci95%=[1.56;7.12]).

**Table 4: Correlation between POLQ expression and clinical status**

| | Pol Q Low (N = 138) | | Pol Q High (N = 65) | | |
|---|---|---|---|---|---|
| | n | % | n | % | p |
| Age (cl) | | | | | p = 0.5589 (chi -2) |
| < 50 | 64 | (46.4) | 33 | (50.8) | |
| ≥ 50 | 74 | (53.6) | 32 | (49.2) | |
| Positive nodes | | | | | p = 0.0172 (chi -2) |
| 1-3 | 94 | (68.1) | 33 | (50.8) | |
| ≥ 3 | 44 | (31.9) | 32 | (49.2) | |
| Histological type | | | | | p = 0.0003 (chi -2) |
| Ductal | 97 | (70.3) | 62 | (95.4) | |
| carcinoma | | | | | |
| Lobular | 21 | (15.2) | 2 | (3.1) | |
| carcinoma | | | | | |
| Other | 20 | (14.5) | 1 | (1.5) | |
| Histological grade | | | | | p < 0.0001 (chi -2) |
| I-II | 89 | (67.4) | 12 | (18.5) | |
| III | 43 | (32.6) | 53 | (81.5) | |
| Missing | 6 | | 0 | | |
| Tumor size | | | | | p = 0.0432 (chi -2) |
| < 2 cm | 40 | (29.6) | 10 | (16.1) | |
| ≥ 2 cm | 95 | (70.4) | 52 | (83.9) | |
| Missing | 3 | | 3 | | |
| Estrogen receptor | | | | | p < 0.0001 (chi -2) |
| Negative | 28 | (20.9) | 31 | (48.4) | |
| Positive | 106 | (79.1) | 33 | (51.6) | |
| Missing | 4 | | 1 | | |
| Progesterone receptor | | | | | p = 0.0034 (chi -2) |
| Negative | 69 | (51.5) | 47 | (73.4) | |
| Positive | 65 | (48.5) | 17 | (26.6) | |
| Missing | 4 | | 1 | | |
| Ki 67 | | | | | p < 0.0001 (chi -2) |
| <20 % | 72 | (66.1) | 19 | (33.9) | |
| >20 % | 37 | (33.9) | 37 | (66.1) | |
| Missing | 29 | | 9 | | |
| HER2 status | | | | | p = 0.0032 (chi -2) |
| Negative | 109 | (89.3) | 41 | (71.9) | |
| Positive | 13 | (10.7) | 16 | (28.1) | |
| Missing | 16 | | 8 | | |
| Molecular sub-type | | | | | p = 0.0422 (chi -2) |
| Triple | 15 | (12.7) | 14 | (25.0) | |
| negative | | | | | |
| Others | 103 | (87.3) | 42 | (75.0) | |
| Missing | 20 | | 9 | | |

**Table 5: Multivariate analysis - cancer specific survival (n = 192)**

| | Initial model ^{a} | | | Final model ^{b} | | |
|---|---|---|---|---|---|---|
| | HR | CI95% | p (Wald) | HR | CI95% | p (Wald) |
| Nb lymph node involved | | | | | | |
| 1-3 | 1 | | | 1 | | |
| > 3 | 2.80 | [1.29; 6.07) | 0.009 | 2.78 | [1.29; 6.01] | 0.009 |

| Histological grade | | | | | | |
|---|---|---|---|---|---|---|
| I - II | 1 | | | | | |
| III | 2.33 | [0.91; 6.01] | 0.079 | NS ^{c} | | |

| Hormone receptors | | | | | | |
|---|---|---|---|---|---|---|
| Negative | 1.54 | [0.71; 3.36] | 0.274 | NS | | |
| Positive | 1 | | | | | |

| Vascular invasion | | | | | | |
|---|---|---|---|---|---|---|
| Absence | 1 | | | | | |
| presence | 1.71 | [0.81; 3.63] | 0.161 | NS | | |

| POLQ expression | | | | | | |
|---|---|---|---|---|---|---|
| Low | 1 | | | | | |
| High | 1.99 | [0.86; 4.61] | 0.109 | 3.33 | [1.56; 7.12] | 0.002 |

| | | | | | | |
|---|---|---|---|---|---|---|
| a. Initial model: including all variables with p < 0.05in univariate analysis. b. Final model: same model after backward stepwise algorithm. c. NS, not significant after stepwise algorithm. HR, hazard ratio; CI confidence interval. | | | | | | |

### 2.3. Ectopic expression of POLQ affects cell cycle and proliferation

The data suggest that either *POLQ* is only a bystander prognosis marker or that it could actively contribute to tumor progression. To evaluate the specific impact of POLQ overexpression on genetic stability and cell proliferation we generated three recombinant *POLQ* clones (Q1, Q2 and Q3) which stably over-express increasing amounts of POLQ protein compared to the endogenous levels displayed by isogenic controls (Fig. 5 A). The over-expression of *POLQ* transcripts in these clones (ranging from 10 to 15-fold) was comparable to that observed in tumors (data not shown). Quantitative FACS analysis after DNA staining (Fig. 5 B) revealed that Q1, Q2 and to a larger extent Q3, significantly accumulated in S phase compared to controls. We also observed a significant accumulation of Q3 cells, which expressed the highest amount of POLQ, in G2/M phase. Consistent with retardation in S phase and G2/M transition, we found that the Q3 cells proliferated more slowly in exponential culture, with an increased doubling time (25.2 h) compared to control cells (22.5 h for CTL1 and 20.2 h for CTL2) or to the other POLQ expressing clones (20.0 h for Q1 and 22.7 h for Q2) (data not shown). Taken together, these results indicate that up-regulation of *POLQ* in human cells affects cell-cycle progression in the absence of external stress.

### 2.4. Ectopic POLQ expression induces DNA break-mediated damage signaling

To investigate the impact of POLQ over-expression on genomic stability, we next performed immunofluorescence assays to detect γ-H2AX, the phosphorylated form of histone H2AX that accumulates at DSB. We observed that the POLQ over-expressing cells clearly showed a significant 2 to 3 fold increase in γ-H2AX foci formation compared to isogenic controls (Fig. 6A, Q1 p<0.002; Q2, p< 0.001; Q3 p<0.003). Immunoblotting analysis with -H2AX antibodies (Fig. 7) confirmed these data. The level of activation of two central transducer kinases in DNA damage signaling, CHK1 and CHK2, was then determined. The level of phosphorylated CHK1 remained unchanged in the presence of high level of *POLQ* (data not shown). In contrast, immunodetection experiments analyzing foci formation of the phosphorylated form of CHK2 revealed a significant activation of the CHK2 kinase in all three cell lines (Q1 p<0.0018; Q2, p< 0.002; Q3 p<0.01) compared to isogenic control cells (Fig. 6B). This data was confirmed by immunoblotting (data not shown). Interestingly, confocal analysis indicated that most of PT68-CHK2 foci in all the POLQ over-expressor cell lines colocalized with γ-H2AX foci (Fig. 6C), showing that activated CHK2 kinase was localized to sites of DNA damage. This suggests that over-expression of POLQ induces the activation of the yH2AX-ATM-CHK2 DNA damage checkpoint.

### 2.5. POLQ over-expressing cells displayed spontaneous chromosome abnormalities

To further investigate the consequences of POLQ over-expression on chromosomal stability, we analyzed metaphase spreads from the Q1, Q2, Q3 clones and isogenic controls. POLQ over-expression resulted in a significant increase in the frequency of cells displaying quadriradials, end-to-end fusions and chromatid breaks (25% of aberrant metaphases were detected in *POLQ* cells versus 15% in controls; Fig. 8). These data indicate that spontaneous chromosome instability occurs in POLQ over-expressing cells, which may be the consequences of the increased DNA breakage observed.

### 2.6. Replication dynamics is perturbed in POLQ-overexpressing cells

A proposed role of POLQ in BER could be that over-expressed POLQ interferes with the BER pathway and leads to an accumulation of endogenous DNA damage (including damage generated by reactive oxygen species). To test this hypothesis, we measured the sensitivity of the POLQ over-expressing cells to methyl-methanesulfonate (MMS) and Nitroso- N-methylurea (MNU) (Fig. 9), both of which induce DNA damage repaired by BER. We found that POLQ over-expression did not significantly affect survival to MMS and MNU treatments, suggesting that defective cell cycle progression, genetic instability as well as activation of the DNA damage checkpoint do not result from a defective BER of endogenous DNA damage in the POLQ over-expressing cells.

The results presented above led us to examine replication dynamics in POLQ overexpressing cells. To assess whether POLQ over-expression affected the rate of replication fork progression, dynamic molecular combing was performed. This method allowed us to determine the polarity of replication forks *in vivo* at the level of individual replicating DNA molecules as well as the distribution of fork velocities (Fig. 10). The median velocity measured in control cells was 1.699 kb/min (n=144) whereas Q2 and Q3 cells displayed a lower velocity of 1.403 kb/min (n=113) and 1.402 kb/min (n=200), respectively. In conclusion, POLQ over-expressing cells displayed a substantial reduction in the overall replication speed, demonstrating that excess POLQ constitutes a potent inducer of replicative stress in human cells. A similar over-expression of either POLB or POLK is sufficient to impede replication fork progression (Chirgwin et al. Biochemistry 18: 5294-5299, 1979).

### 2.7. POLQ-overexpressing cells are hypersensitive to a Chk2 inhibitor

MDA-MB231 and MCF7 breast cancer cells (ATCC) were grown in RPMI 1640+ glutamax medium. A 13.8 mM Chk2 inhibitor II Hydrate (Sigma RefC3742) stock solution was prepared by diluting the agent in DMSO (Sigma) and stored at -20°C. This solution was diluted in culture medium to give as indicated the final drug concentration. Cells were exposed to different concentrations ranging from 1µM to 150µM during 16 hours. Cells were washed with PBS buffer, fresh medium was added and cells were incubated for seven more days. Cells were fixed with NaCl 0.9% and stained with crystal violet (Sigma C3886). Clones containing more than 20 cells were counted. For the clonogenic assay 400 cells were seeded in six-well plates and incubated overnight. The POLQ expression was measured by real-time PCR as described above.

Cells overexpressing POLQ accumulate DNA damage. Said damages trigger the ATM-dependent DNA damage checkpoint through activation of the Chk2 protein kinase, eventually leading to cell-cycle arrest. This suggested to us that POLQ-overexpressing cells would be hypersensitive to inhibitors of DNA damage signaling. In order to test this hypothesis, the viability of the MCF7 and MDA-MB231 cells was determined in the presence of increasing amounts of a Chk2 inhibitor. In parallel, POLQ expression was assayed in each cell line.

As shown on figure 12, MCF7 which express more than twice as much POLQ RNA than MDA-MB231 are hypersensitive to the CHK2 inhibitor. There is thus a correlation between the expression of POLQ and the sensitivity to the inhibition of the DNA damage signaling pathway.

## Claims

1. A method for prognosing bad survival of a patient suffering from a breast cancer, comprising:
a) measuring in vitro the expression level of the POLQ gene and/or one or several of its isoforms, and the expression level of a control gene in a breast cancer sample of said patient,
b) calculating for said POLQ gene and/or isoform an expression level ratio of the expression level of POLQ and/or one or several of its isoforms to the expression of the said control gene in said patient breast cancer sample
c) comparing said gene expression level ratio to a corresponding threshold value, and
d) prognosing bad survival if the said ratio is superior to its corresponding threshold value.

2. The method of claim 1, wherein the patient has a high number of metastatic lymph nodes.

3. The method of claim 1, wherein the breast cancer expresses a wild-type p53 cDNA

4. A method for diagnosing genetic instability in a breast cancer in a patient from a breast cancer sample of said patient, comprising:
a) measuring in vitro the expression level of the POLQ gene and one or several of its isoforms, and the expression level of a control gene in said patient breast cancer sample
b) calculating for said POLQ gene and/or isoform an expression level ratio of the expression level of POLQ and/or one or several of its isoforms to the expression of the said control gene in said patient breast cancer sample
c) comparing said gene expression level ratio to a corresponding threshold value, and
d) diagnosing breast cancer genetic instability if the said ratio is superior to its corresponding threshold value.

5. The method of any one of claims 1-4, wherein said control gene is a housekeeping gene.

6. The method of claim 5, wherein said housekeeping gene is a gene selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS.

7. The method of claim 6, wherein said gene is IPO8 or HMBS.

8. The method of any one of claims 1-7, wherein said expression level is measured at the mRNA level.

9. The method of claim 8, wherein said expression level is measured using quantitative PCR or microarray technology.

10. The method of any one of claims 1-9, wherein said expression level is measured at the protein level.

11. The method of claim 10, wherein said expression level is measured using specific antibodies.

12. The method of any one of claims 1-11, wherein said threshold value is 15.8

13. Use of a microarray comprising at most 500 probes, at least one of which specifically hybridizes to POLQ, and at least one other probe specifically hybridizes to a gene selected in the group consisting of the IPO8 gene and the HBMS gene in the method of any of claims 1 - 12.

14. Use of a kit, comprising a microarray, said microarray comprising at most 500 probes, at least one of which specifically hybridizes to POLQ, and at least one other probe specifically hybridizes to a gene selected from the group consisting of the IPO8 gene and the HBMS gene or amplification primers for POLQ, said kit further comprising amplification primers specific for a gene selected from the group consisting of the IPO8 gene and the HBMS gene, in the method of any of claims 1 - 12.

15. A method for prognosing the efficiency of radiotherapy or DNA repair, DNA damage signaling or nucleotide metabolism inhibitors in the treatment of breast cancer in a patient, comprising:
a) diagnosing or not genetic instability in said breast cancer in said patient using the method of claim 4, and
b) prognosing efficiency of radiotherapy or DNA repair inhibitors if genetic instability has been diagnosed in step a).

## Patentansprüche

1. Verfahren zur Stellung einer schlechten Überlebensprognose eines an Brustkrebs leidenden Patienten, umfassend:
a) In-vitro-Messung des Expressionslevels des POLQ-Gens und/oder von einem oder mehrerer seiner Isoformen, und des Expressionslevels eines Kontrollgens in einer Brustkrebsprobe des Patienten,
b) Berechnen für das POLQ-Gen und/oder der Isoform eines Expressionslevelverhältnisses des Expressionslevels von POLQ und/oder einem oder mehrerer seiner Isoformen zur Expression des Kontrollgens in der Brustkrebsprobe des Patienten,
c) Vergleichen des Genexpressionslevelverhältnisses mit einem entsprechenden Schwellenwert, und
d) Stellen einer schlechten Überlebensprognose, wenn das Verhältnis größer ist als sein entsprechender Schwellenwert.

2. Verfahren nach Anspruch 1, wobei der Patient eine große Zahl an metastatischen Lymphknoten aufweist.

3. Verfahren nach Anspruch 1, wobei der Brustkrebs eine Wildtyp-p53-cDNA exprimiert.

4. Verfahren zur Diagnose von genetischer Instabilität in einem Brustkrebs in einem Patienten anhand einer Brustkrebsprobe des Patienten, umfassend:
a) In-vitro-Messung des Expressionslevels des POLQ-Gens und von einem oder mehrerer seiner Isoformen, und des Expressionslevels eines Kontrollgens in der Brustkrebsprobe des Patienten,
b) Berechnen für das POLQ-Gen und/oder der Isoform eines Expressionslevelverhältnisses des Expressionslevels von POLQ und/oder einem oder mehrerer seiner Isoformen zur Expression des Kontrollgens in der Brustkrebsprobe des Patienten,
c) Vergleichen des Genexpressionslevelverhältnisses mit einem entsprechenden Schwellenwert, und
d) Diagnostizieren von genetischer Instabilität in dem Brustkrebs, wenn das Verhältnis größer ist als sein entsprechender Schwellenwert.

5. Verfahren nach einem der Ansprüche 1 bis 4, wobei das Kontrollgen ein Haushaltsgen ist.

6. Verfahren nach Anspruch 5, wobei das Haushaltsgen ein Gen ist, das ausgewählt ist aus der Gruppe bestehend aus B2M, TFRC, YWHAZ, RPLO, 18S, GUSB, UBC, TBP, GAPDH, PPIA, POLR2A, ACTB, PGK1, HPRT1, IP08 und HMBS.

7. Verfahren nach Anspruch 6, wobei das Gen IP08 oder HMBS ist.

8. Verfahren nach einem der Ansprüche 1 bis 7, wobei der Expressionslevel auf der mRNA-Ebene gemessen wird.

9. Verfahren nach Anspruch 8, wobei der Expressionslevel unter Verwendung von quantitativer PCR oder Mikroarray-Technologie gemessen wird.

10. Verfahren nach einem der Ansprüche 1 bis 9, wobei der Expressionslevel auf der Proteinebene gemessen wird.

11. Verfahren nach Anspruch 10, wobei der Expressionslevel unter Verwendung spezifischer Antikörper gemessen wird.

12. Verfahren nach einem der Ansprüche 1 bis 11, wobei der Schwellenwert 15,8 ist.

13. Verwendung eines Mikroarrays mit maximal 500 Sonden, von denen mindestens eine spezifisch an POLQ hybridisiert und mindestens eine andere Sonde spezifisch an ein Gen hybridisiert, das ausgewählt ist aus der Gruppe bestehend aus dem IP08-Gen und dem HBMS-Gen, in einem Verfahren nach einem der Ansprüche 1 bis 12.

14. Verwendung eines Kits umfassend ein Mikroarray, wobei das Mikroarray maximal 500 Sonden umfasst, von denen mindestens eines spezifisch an POLQ hybridisiert und mindestens eine andere Sonde spezifisch an ein Gen hybridisiert, das ausgewählt ist aus der Gruppe bestehend aus dem IP08-Gen und dem HBMS-Gen, oder Amplifikations-Primer für POLQ, wobei das Kit ferner Amplifikations-Primer umfasst, die spezifisch sind für ein Gen ausgewählt aus der Gruppe bestehend aus dem IP08-Gen und dem HBMS-Gen sind, in einem Verfahren nach einem der Ansprüche 1 bis 12.

15. Verfahren zur Prognose der Effizienz von Strahlentherapie oder von Inhibitoren der DNA-Reparatur, der DNA-Schaden-Signalisierung oder des Nukleotid-Metabolismus bei der Behandlung von Brustkrebs bei einem Patienten, umfassend:
a) Diagnostizieren oder nicht von genetischer Instabilität in dem Brustkrebs in dem Patienten unter Verwendung des Verfahrens nach Anspruch 4, und
b) Prognostizieren der Effizienz von Strahlentherapie oder DNA-Reparatur-Inhibitoren, wenn in Schritt a) genetische Instabilität diagnostiziert wurde.

## Revendications

1. Méthode de pronostic de survie défavorable d'un patient souffrant d'un cancer du sein, comprenant :
a) la mesure du taux d'expression *in vitro* du gène POLQ et/ou d'une ou plusieurs de ses isoformes, et le taux d'expression d'un gène témoin dans un échantillon de cancer du sein chez ledit patient,
b) le calcul pour ledit gène POLQ et/ou ladite isoforme d'un rapport de taux d'expression du taux d'expression de POLQ et/ou d'une ou plusieurs de ses isoformes à l'expression dudit gène témoin chez ledit échantillon de cancer du sein du patient
c) la comparaison dudit rapport du taux d'expression de gène à une valeur seuil correspondante, et
d) le pronostic de survie défavorable si ledit rapport est supérieur à sa valeur seuil correspondante.

2. Méthode selon la revendication 1, dans laquelle le patient a un nombre élevé de ganglions lymphatiques métastasiques.

3. Méthode selon la revendication 1, dans laquelle le cancer du sein exprime un ADNc de type sauvage de p53.

4. Méthode de diagnostic de l'instabilité génétique dans un cancer du sein chez un patient à partir d'un échantillon de cancer du sein dudit patient, comprenant :
a) la mesure du taux d'expression *in vitro* du gène POLQ et d'une ou plusieurs de ses isoformes, et le taux d'expression d'un gène témoin dans ledit échantillon de cancer du sein chez ledit patient,
b) le calcul pour ledit gène POLQ et/ou ladite isoforme d'un rapport de taux d'expression du taux d'expression de POLQ et/ou d'une ou plusieurs de ses isoformes à l'expression dudit gène témoin chez ledit échantillon de cancer du sein du patient
c) la comparaison dudit rapport du taux d'expression de gène à une valeur seuil correspondante, et
d) le diagnostic de l'instabilité génétique du cancer du sein si ledit rapport est supérieur à sa valeur seuil correspondante.

5. Méthode selon l'une quelconque des revendications 1 à 4, dans laquelle ledit gène témoin est un gène ménager.

6. Méthode selon la revendication 5, dans laquelle ledit gène ménager est un gène choisi dans le groupe comprenant B2M, TFRC, YWHAZ, RPLO, 185, GUSB, UBC, TBP, GAPDH, PPIA, POLRZA, ACTB, PGK1, HPRT1, IPO8 et HMBS.

7. Méthode selon la revendication 6, dans laquelle ledit gène est IPO8 ou HMBS.

8. Méthode selon l'une quelconque des revendications 1 à 7, dans laquelle ledit taux d'expression est mesuré au niveau de l'ARNm.

9. Méthode selon la revendication 8, dans laquelle ledit taux d'expression est mesuré en utilisant une PCR quantitative ou une technologie à micropuce.

10. Méthode selon l'une quelconque des revendications 1 à 9, dans laquelle ledit taux d'expression est mesuré au niveau des protéines.

11. Méthode selon la revendication 10, dans laquelle ledit taux d'expression est mesuré en utilisant des anticorps spécifiques.

12. Méthode selon l'une quelconque des revendications 1 à 11, dans laquelle ladite valeur seuil est de 15,8.

13. Utilisation d'une micropuce comprenant au plus 500 sondes, dont au moins l'une s'hybride spécifiquement à POLQ, et au moins une autre sonde s'hybride spécifiquement à un gène choisi dans le groupe comprenant le gène IPO8 et le gène HBMS dans la méthode selon l'une quelconque des revendications 1 à 12.

14. Utilisation d'un kit comprenant une micropuce, ladite micropuce comprenant au plus 500 sondes, dont au moins l'une s'hybride spécifiquement à POLQ, et au moins une autre sonde s'hybride spécifiquement à un gène choisi dans le groupe comprenant le gène IPO8 et le gène HBMS ou des amorces d'amplification pour POLQ, ledit kit comprenant en outre des amorces d'amplification spécifiques d'un gène choisi dans le groupe comprenant le gène IPO8 et le gène HBMS, dans la méthode selon l'une quelconque des revendications 1 à 12.

15. Méthode de pronostic de l'efficacité d'une radiothérapie ou d'une réparation de l'ADN, du signalement d'un dommage de l'ADN ou d'inhibiteurs du métabolisme des nucléotides dans le traitement d'un cancer du sein chez un patient, comprenant :
a) le diagnostic ou non de l'instabilité génétique dans ledit cancer du sein chez ledit patient en utilisant la méthode selon la revendication 4, et
b) le pronostic de l'efficacité d'une radiothérapie ou d'inhibiteurs de la réparation de l'ADN si l'instabilité génétique a été diagnostiquée à l'étape a).


### SEQUENCE LISTING

<110> Centre National de la Recherche Scientifique (CNRS) Universite Paul Sabatier Toulouse III Institut Claudius Regaud
<120> SIGNATURE FOR THE DIAGNOSIS OF BREAST CANCER AGGRESSIVENESS AND
   GENETIC INSTABILITY
<130> D28090
<150> EP09306096.0
   <151> 2009-11-13
<160> 15
<170> PatentIn version 3.3
<210> 1
   <211> 25
   <212> DNA
   <213> artificial sequence
<220>
   <223> IPO8 primer
<400> 1
   aggggaattg atcagtgcat tccac 25
<210> 2
   <211> 25
   <212> DNA
   <213> artificial sequence
<220>
   <223> HMBS primer
<400> 2
   gcggctgcaa cggcggaaga aaaca 25
<210> 3
   <211> 25
   <212> DNA
   <213> artificial sequence
<220>
   <223> POLA primer
<400> 3
   tacaaccaac caggtgtggt atttc 25
<210> 4
   <211> 25
   <212> DNA
   <213> artificial sequence
<220>
   <223> POLD1 primer
<400> 4
   ctgtttgaag cgggatggat ggcaa 25
<210> 5
   <211> 25
   <212> DNA
   <213> artificial sequence
<220>
   <223> POLE primer
<400> 5
   ctttgaagag gtgtgtgatg agatt 25
<210> 6
   <211> 25
   <212> DNA
   <213> artificial sequence
<220>
   <223> REV1L primer
<400> 6
   gggaaacatg gggtgggtat atggc 25
<210> 7
   <211> 25
   <212> DNA
   <213> artificial sequence
<220>
   <223> POLH primer
<400> 7
   tcacacaata aggtcctggc aaaac 25
<210> 8
   <211> 25
   <212> DNA
   <213> artificial sequence
<220>
   <223> POLM primer
<400> 8
   gcagaaagcg gggctccagc accac 25
<210> 9
   <211> 25
   <212> DNA
   <213> artificial sequence
<220>
   <223> POLK primer
<400> 9
   gccacgaagg ggtccagatt ttatg 25
<210> 10
   <211> 25
   <212> DNA
   <213> artificial sequence
<220>
   <223> REV3L primer
<400> 10
   aaaagcccag ggagattggt ggacg 25
<210> 11
   <211> 25
   <212> DNA
   <213> artificial sequence
<220>
   <223> POLL primer
<400> 11
   gattgagcag acagtccaga aagca 25
<210> 12
   <211> 25
   <212> DNA
   <213> artificial sequence
<220>
   <223> POLB primer
<400> 12
   gagttagtgg cattggtcca tctgc 25
<210> 13
   <211> 25
   <212> DNA
   <213> artificial sequence
<220>
   <223> POLN primer
<400> 13
   tggagcaggg aaggagcggc tggct 25
<210> 14
   <211> 25
   <212> DNA
   <213> artificial sequence
<220>
   <223> POLI primer
<400> 14
   ccagctcgca gggagttcat gatca 25
<210> 15
   <211> 25
   <212> DNA
   <213> artificial sequence
<220>
   <223> POLQ primer
<400> 15
   gcctttccca ggtggttcaa tactg 25